# EUROPEAN PATENT APPLICATION

(11) **EP 2 436 775 A1**
(43) Date of publication of application: **04.04.2012**
(21) Application number: 10780676.2
(22) Date of filing: 25.05.2010
(51) Int. Cl.: C12Q 1/02, C12N 5/0735, G01N 33/15, G01N 33/50, C12N 15/09, C12Q 1/68

(54) **METHOD FOR SCREENING OF REGENERATIVE MEDICINE**

(30) Priority: 26.05.2009 JP 2009126635
(71) Applicant: Takeda Pharmaceutical Company Limited, Osaka-shi, Osaka 541-0045 (JP)
(72) Inventor: HOSOYA, Masaki, Fujisawa, Kanagawa 251-0012 (JP); KUNISADA, Yuya, Fujisawa, Kanagawa 251-0012 (JP); SHOJI, Masanobu, Fujisawa, Kanagawa 251-0012 (JP)
(74) Representative: König, Gregor Sebastian
(86) International application number: PCT/JP2010/059169
(87) International publication number: WO 2010/137722

(57) **Abstract**

A method for screening for a substance capable of regulating the regeneration, proliferation or differentiation of a cell or an organ, which comprises the steps of: (1) allowing a cell having a regenerative, proliferative or differentiative capability to form an embryoid body; (2) treating the embryoid body produced in step (1) with a digestive enzyme to prepare single cells from the embryoid body; (3) seeding the cells prepared in step (2) onto an adhesive plate, and adding a candidate substance to the plate to perform adhesion culturing of the cells on the plate; (4) conducting quantitative and simultaneous analysis of the levels of expression of at least two types of genes involved in the regeneration, proliferation or differentiation of cells after the adhesion culturing of step (3); and (5) evaluating the influence of the candidate substance on the regeneration, proliferation or differentiation of cells based on the results of the quantitative analysis obtained in step (4).

SEQUENCE LISTING

<110>  TAKEDA PHARMACEUTICAL COMPANY LIMITED

<120>  METHOD OF SCREENING FOR REGENERATION MEDICINE

<130>  PCT10-0015

<150>  JP2009-126635
<151>  2009-05-26

<160>  138

<170>  PatentIn version 3.3

<210>  1
<211>  20
<212>  DNA
<213>  Artificial

<220>
<223>  Primer

<400>  1
ttcctacccc caatgtgtcc                                                    20

<210>  2
<211>  24
<212>  DNA
<213>  Artificial

<220>
<223>  Primer

<400>  2
tgtcatcata cttggcaggt ttct                                               24

<210>  3
<211>  22
<212>  DNA
<213>  Artificial

<220>
<223>  Probe

<400> 3
tcgtggatct gacgtgccgc ct 22

<210> 4
<211> 24
<212> DNA
<213> Artificial

<220>
<223> Primer

<400> 4
aacgagatga ttgtgaccaa gaac 24

<210> 5
<211> 23
<212> DNA
<213> Artificial

<220>
<223> Primer

<400> 5
aggcctgaca catttacctt cag 23

<210> 6
<211> 17
<212> DNA
<213> Artificial

<220>
<223> Probe

<400> 6
caggaggatg ttcccgg 17

<210> 7
<211> 19
<212> DNA
<213> Artificial

<220>
<223> Primer

<400> 7
   ttgtgaatgt cccacccca 19

<210> 8
<211> 23
<212> DNA
<213> Artificial

<220>
<223> Primer

<400> 8
ggtcccatac tggtaggaat cca 23

<210> 9
<211> 25
<212> DNA
<213> Artificial

<220>
<223> Probe

<400> 9
cggtgagaaa gccttgatct cgcct 25

<210> 10
<211> 18
<212> DNA
<213> Artificial

<220>
<223> Primer

<400> 10
ctcggcctct tccacgaa 18

<210> 11
<211> 22
<212> DNA
<213> Artificial

<220>

<223>  Primer

<400>  11
atctgcacaa cgcagagcta ag                                              22

<210>  12
<211>  19
<212>  DNA
<213>  Artificial

<220>
<223>  Probe

<400>  12
tccaagactt gcctagcat                                                  19

<210>  13
<211>  19
<212>  DNA
<213>  Artificial

<220>
<223>  Primer

<400>  13
ccatgcaccg ctacgacat                                                  19

<210>  14
<211>  17
<212>  DNA
<213>  Artificial

<220>
<223>  Primer

<400>  14
gaaggcgacg cgctcat                                                    17

<210>  15
<211>  17
<212>  DNA
<213>  Artificial

<220>
<223>  Probe

<400>  15
ctccaactct cagggct                                                    17


<210>  16
<211>  19
<212>  DNA
<213>  Artificial

<220>
<223>  Primer

<400>  16
aaggcctcga agcgacaga                                                  19


<210>  17
<211>  20
<212>  DNA
<213>  Artificial

<220>
<223>  Primer

<400>  17
cagtttgcca agctgctgaa                                                 20


<210>  18
<211>  18
<212>  DNA
<213>  Artificial

<220>
<223>  Probe

<400>  18
cacctttcca aagagaac                                                   18


<210>  19
<211>  19
<212>  DNA
<213>  Artificial

<220>
<223> Primer

<400> 19
aagcggtggc agaaaaacc 19

<210> 20
<211> 21
<212> DNA
<213> Artificial

<220>
<223> Primer

<400> 20
gtgctgagcc cttctgaatc a 21

<210> 21
<211> 19
<212> DNA
<213> Artificial

<220>
<223> Probe

<400> 21
tggttgaaga ctagcaatg 19

<210> 22
<211> 25
<212> DNA
<213> Artificial

<220>
<223> Primer

<400> 22
ggatctgagc gatctgactc tgtag 25

<210> 23
<211> 20
<212> DNA

<213>  Artificial

<220>
<223>  Primer

<400>  23
aggcgctgga acagagattg                                                    20


<210>  24
<211>  15
<212>  DNA
<213>  Artificial

<220>
<223>  Probe

<400>  24
cagccagctg gaact                                                         15


<210>  25
<211>  22
<212>  DNA
<213>  Artificial

<220>
<223>  Primer

<400>  25
gccaagtgaa aaccaggaca aa                                                 22


<210>  26
<211>  28
<212>  DNA
<213>  Artificial

<220>
<223>  Primer

<400>  26
tgtatcgact aaagtgaaac tccttctc                                           28


<210>  27
<211>  18

<212>  DNA
<213>  Artificial

<220>
<223>  Probe

<400>  27
tacacagacc atcagcgg                                                    18


<210>  28
<211>  21
<212>  DNA
<213>  Artificial

<220>
<223>  Primer

<400>  28
cccattctgg acatggtgaa a                                                21


<210>  29
<211>  24
<212>  DNA
<213>  Artificial

<220>
<223>  Primer

<400>  29
agcacgcaga aaccataaat taaa                                             24


<210>  30
<211>  15
<212>  DNA
<213>  Artificial

<220>
<223>  Probe

<400>  30
ccaggtctcg ggtct                                                       15


<210>  31

<211>  20
<212>  DNA
<213>  Artificial

<220>
<223>  Primer

<400>  31
gcttccgctg tccagagact                                                    20


<210>  32
<211>  20
<212>  DNA
<213>  Artificial

<220>
<223>  Primer

<400>  32
tgccagcaga ttccatacca                                                    20


<210>  33
<211>  16
<212>  DNA
<213>  Artificial

<220>
<223>  Probe

<400>  33
tcttccagcc ctcttt                                                        16


<210>  34
<211>  21
<212>  DNA
<213>  Artificial

<220>
<223>  Primer

<400>  34
cttccagcca tctttcattg g                                                  21

<210> 35
<211> 24
<212> DNA
<213> Artificial

<220>
<223> Primer

<400> 35
tgatgctgtt ataggtggtt tcgt 24


<210> 36
<211> 15
<212> DNA
<213> Artificial

<220>
<223> Probe

<400> 36
atggagtcag cgggc 15


<210> 37
<211> 23
<212> DNA
<213> Artificial

<220>
<223> Primer

<400> 37
caatcaggcc tggaaataca ttg 23


<210> 38
<211> 19
<212> DNA
<213> Artificial

<220>
<223> Primer

<400> 38
tccgcggctc caagtagtt 19

<210> 39
<211> 21
<212> DNA
<213> Artificial

<220>
<223> Probe

<400> 39
tgacgaatga gattttcact c 21


<210> 40
<211> 22
<212> DNA
<213> Artificial

<220>
<223> Primer

<400> 40
acattgagat tgccgcttac ag 22


<210> 41
<211> 20
<212> DNA
<213> Artificial

<220>
<223> Primer

<400> 41
accaaagcca atccgacact 20


<210> 42
<211> 15
<212> DNA
<213> Artificial

<220>
<223> Probe

<400> 42
aagctcctgg aaggc 15

<210> 43
<211> 22
<212> DNA
<213> Artificial

<220>
<223> Primer

<400> 43
cgcacgacat ctaggactga gt 22


<210> 44
<211> 21
<212> DNA
<213> Artificial

<220>
<223> Primer

<400> 44
cctattcagg cccgacaact t 21


<210> 45
<211> 18
<212> DNA
<213> Artificial

<220>
<223> Probe

<400> 45
caccactctg accaaaga 18


<210> 46
<211> 19
<212> DNA
<213> Artificial

<220>
<223> Primer

<400> 46

tcgcccacct acagcatgt                                                    19

<210>  47
<211>  19
<212>  DNA
<213>  Artificial

<220>
<223>  Primer

<400>  47
tgaccacaga gcccatgga                                                    19

<210>  48
<211>  15
<212>  DNA
<213>  Artificial

<220>
<223>  Probe

<400>  48
tactcgcagc agggc                                                        15

<210>  49
<211>  18
<212>  DNA
<213>  Artificial

<220>
<223>  Primer

<400>  49
cagcaggtgc cccgacta                                                     18

<210>  50
<211>  22
<212>  DNA
<213>  Artificial

<220>
<223>  Primer

<400>  50
ggaagtcgct tcatgtgaga ga                                                22

<210>  51
<211>  15
<212>  DNA
<213>  Artificial

<220>
<223>  Probe

<400>  51
ccgttggcgt gagga                                                        15

<210>  52
<211>  19
<212>  DNA
<213>  Artificial

<220>
<223>  Primer

<400>  52
cgctgcccga atccttaag                                                    19

<210>  53
<211>  16
<212>  DNA
<213>  Artificial

<220>
<223>  Primer

<400>  53
agtgcccgcg ctgtca                                                       16

<210>  54
<211>  18
<212>  DNA
<213>  Artificial

<220>
<223>  Probe

<400>  54
ccctctccaa gacatata                                                    18


<210>  55
<211>  25
<212>  DNA
<213>  Artificial

<220>
<223>  Primer

<400>  55
agtgccaaat ttacggatga ctgta                                            25


<210>  56
<211>  27
<212>  DNA
<213>  Artificial

<220>
<223>  Primer

<400>  56
ggacgcatag gtattatagc tgttttc                                          27


<210>  57
<211>  15
<212>  DNA
<213>  Artificial

<220>
<223>  Probe

<400>  57
cagggagaga ttcca                                                       15


<210>  58
<211>  17
<212>  DNA
<213>  Artificial

<220>

<223>  Primer

<400>  58
gcctgtgccc gaacctt                                                    17


<210>  59
<211>  21
<212>  DNA
<213>  Artificial

<220>
<223>  Primer

<400>  59
gccagactct tggtgcttgt g                                               21


<210>  60
<211>  14
<212>  DNA
<213>  Artificial

<220>
<223>  Probe

<400>  60
cccgcatgtc cttg                                                       14


<210>  61
<211>  23
<212>  DNA
<213>  Artificial

<220>
<223>  Primer

<400>  61
gttcaagatt ggctcccaag aat                                             23


<210>  62
<211>  23
<212>  DNA
<213>  Artificial

<220>
<223> Primer

<400> 62
gcaggacttg gcataataaa acg 23


<210> 63
<211> 20
<212> DNA
<213> Artificial

<220>
<223> Probe

<400> 63
ttctgtcaag acaccatgat 20


<210> 64
<211> 23
<212> DNA
<213> Artificial

<220>
<223> Primer

<400> 64
cagggttcct tgttggagtc ata 23


<210> 65
<211> 21
<212> DNA
<213> Artificial

<220>
<223> Primer

<400> 65
acggctgtag agcgagtgtt g 21


<210> 66
<211> 23
<212> DNA
<213> Artificial

<220>
<223>  Probe

<400>  66
aagtgtatat actcacactg atc                                                23

<210>  67
<211>  24
<212>  DNA
<213>  Artificial

<220>
<223>  Primer

<400>  67
ctatcacaag atgaacggca tcaa                                               24

<210>  68
<211>  21
<212>  DNA
<213>  Artificial

<220>
<223>  Primer

<400>  68
acagcgtggt ggtggtagtc t                                                  21

<210>  69
<211>  14
<212>  DNA
<213>  Artificial

<220>
<223>  Probe

<400>  69
ccgcctgtcc gctt                                                          14

<210>  70
<211>  20
<212>  DNA

<213>  Artificial

<220>
<223>  Primer

<400>  70
tggagctgcc ccagtatacg                                                    20


<210>  71
<211>  20
<212>  DNA
<213>  Artificial

<220>
<223>  Primer

<400>  71
gccgccaaca gtgaagatgt                                                    20


<210>  72
<211>  17
<212>  DNA
<213>  Artificial

<220>
<223>  Probe

<400>  72
catcgactcg cttgttc                                                       17


<210>  73
<211>  19
<212>  DNA
<213>  Artificial

<220>
<223>  Primer

<400>  73
gggcgtcaag tgcttgttg                                                     19


<210>  74
<211>  22

<212>  DNA
<213>  Artificial

<220>
<223>  Primer

<400>  74
gacgtcgttt ccagatgttt cc                                                22


<210>  75
<211>  17
<212>  DNA
<213>  Artificial

<220>
<223>  Probe

<400>  75
agggatggag tgacttt                                                      17


<210>  76
<211>  20
<212>  DNA
<213>  Artificial

<220>
<223>  Primer

<400>  76
gagcgtggct attccttcgt                                                   20


<210>  77
<211>  20
<212>  DNA
<213>  Artificial

<220>
<223>  Primer

<400>  77
tctttgatgt cgcgcacaat                                                   20


<210>  78

<211>  16
<212>  DNA
<213>  Artificial

<220>
<223>  Probe

<400>  78
accacagctg aacgtg                                                    16


<210>  79
<211>  18
<212>  DNA
<213>  Artificial

<220>
<223>  Primer

<400>  79
taagaaggcg gcggaaga                                                  18


<210>  80
<211>  21
<212>  DNA
<213>  Artificial

<220>
<223>  Primer

<400>  80
tgcagtgaca ccagctcatc t                                              21


<210>  81
<211>  14
<212>  DNA
<213>  Artificial

<220>
<223>  Probe

<400>  81
cggagcaagc agct                                                      14

<210> 82
<211> 17
<212> DNA
<213> Artificial

<220>
<223> Primer

<400> 82
agctgcagcc gggttct 17


<210> 83
<211> 20
<212> DNA
<213> Artificial

<220>
<223> Primer

<400> 83
ttctccagct ggtgccagtt 20


<210> 84
<211> 20
<212> DNA
<213> Artificial

<220>
<223> Probe

<400> 84
agaaggtcaa tgagtcaact 20


<210> 85
<211> 19
<212> DNA
<213> Artificial

<220>
<223> Primer

<400> 85
catccctgtt gcccagctt 19

<210> 86
<211> 19
<212> DNA
<213> Artificial

<220>
<223> Primer

<400> 86
tgccctttcc gttgttgtc 19

<210> 87
<211> 15
<212> DNA
<213> Artificial

<220>
<223> Probe

<400> 87
tgagcatgcc ctctg 15

<210> 88
<211> 21
<212> DNA
<213> Artificial

<220>
<223> Primer

<400> 88
gctctggacc tggatgattt g 21

<210> 89
<211> 21
<212> DNA
<213> Artificial

<220>
<223> Primer

<400> 89
acaattcttg gaggcgagga a 21

<210> 90
<211> 15
<212> DNA
<213> Artificial

<220>
<223> Probe

<400> 90
tcctaccagg tggcc 15


<210> 91
<211> 23
<212> DNA
<213> Artificial

<220>
<223> Primer

<400> 91
cttcagatca actttgccag ctt 23


<210> 92
<211> 20
<212> DNA
<213> Artificial

<220>
<223> Primer

<400> 92
attcctcctc cggcatgagt 20


<210> 93
<211> 18
<212> DNA
<213> Artificial

<220>
<223> Probe

<400> 93

ctgagattgc ccagagtg                                                    18


<210>  94
<211>  22
<212>  DNA
<213>  Artificial

<220>
<223>  Primer

<400>  94
atggaaatcc catcaccatc tt                                              22


<210>  95
<211>  18
<212>  DNA
<213>  Artificial

<220>
<223>  Primer

<400>  95
cgccccactt gattttgg                                                    18


<210>  96
<211>  14
<212>  DNA
<213>  Artificial

<220>
<223>  Probe

<400>  96
caggagcgag atcc                                                        14


<210>  97
<211>  21
<212>  DNA
<213>  Artificial

<220>
<223>  Primer

<400> 97
tcagcattca gtgtgggatt g 21

<210> 98
<211> 20
<212> DNA
<213> Artificial

<220>
<223> Primer

<400> 98
ggtaaagcga atgggcatgt 20

<210> 99
<211> 19
<212> DNA
<213> Artificial

<220>
<223> Probe

<400> 99
agacttacgt tactatccc 19

<210> 100
<211> 18
<212> DNA
<213> Artificial

<220>
<223> Primer

<400> 100
tcagggctgc cagtttcg 18

<210> 101
<211> 24
<212> DNA
<213> Artificial

<220>
<223> Primer

<400>  101
gcttttggca tactctgtga tctc                                              24


<210>  102
<211>  15
<212>  DNA
<213>  Artificial

<220>
<223>  Probe

<400>  102
cgtggaggct gtgca                                                        15


<210>  103
<211>  18
<212>  DNA
<213>  Artificial

<220>
<223>  Primer

<400>  103
attgaccacg ggcaccat                                                     18


<210>  104
<211>  17
<212>  DNA
<213>  Artificial

<220>
<223>  Primer

<400>  104
ctccaccgcc tcatgca                                                      17


<210>  105
<211>  17
<212>  DNA
<213>  Artificial

<220>

<223>  Probe

<400>  105
aaggaaaagc caagcag                                                    17


<210>  106
<211>  17
<212>  DNA
<213>  Artificial

<220>
<223>  Primer

<400>  106
cccgtggcct tcaaggt                                                    17


<210>  107
<211>  24
<212>  DNA
<213>  Artificial

<220>
<223>  Primer

<400>  107
tgacagtaac cacagtccca tctg                                            24


<210>  108
<211>  16
<212>  DNA
<213>  Artificial

<220>
<223>  Probe

<400>  108
tagccctcgg agaggt                                                     16


<210>  109
<211>  18
<212>  DNA
<213>  Artificial

<220>
<223> Primer

<400> 109
ccccactcag cccagtgt 18

<210> 110
<211> 22
<212> DNA
<213> Artificial

<220>
<223> Primer

<400> 110
accagacatg cctcttgtcc tt 22

<210> 111
<211> 19
<212> DNA
<213> Artificial

<220>
<223> Probe

<400> 111
ccagaagaac tggtacatc 19

<210> 112
<211> 22
<212> DNA
<213> Artificial

<220>
<223> Primer

<400> 112
ctgtacctgc acagcctcat ct 22

<210> 113
<211> 22
<212> DNA
<213> Artificial

<220>
<223>  Primer

<400>  113
tgaagcccca caggtacttc tt                                                22

<210>  114
<211>  18
<212>  DNA
<213>  Artificial

<220>
<223>  Probe

<400>  114
catggccttc ttctcaga                                                     18

<210>  115
<211>  20
<212>  DNA
<213>  Artificial

<220>
<223>  Primer

<400>  115
ttcctacccc caatgtgtcc                                                   20

<210>  116
<211>  24
<212>  DNA
<213>  Artificial

<220>
<223>  Primer

<400>  116
tgtcatcata cttggcaggt ttct                                              24

<210>  117
<211>  22
<212>  DNA

<213>  Artificial

<220>
<223>  Probe

<400>  117
tcgtggatct gacgtgccgc ct                                                22


<210>  118
<211>  21
<212>  DNA
<213>  Artificial

<220>
<223>  Primer

<400>  118
cttccagcca tctttcattg g                                                 21


<210>  119
<211>  24
<212>  DNA
<213>  Artificial

<220>
<223>  Primer

<400>  119
tgatgctgtt ataggtggtt tcgt                                              24


<210>  120
<211>  15
<212>  DNA
<213>  Artificial

<220>
<223>  Probe

<400>  120
atggagtcag cgggc                                                        15


<210>  121
<211>  24

<212>  DNA
<213>  Artificial

<220>
<223>  Primer

<400>  121
ggagaagatc tggcaccata catt                                              24


<210>  122
<211>  19
<212>  DNA
<213>  Artificial

<220>
<223>  Primer

<400>  122
agcagggttg ggtgttcct                                                    19


<210>  123
<211>  22
<212>  DNA
<213>  Artificial

<220>
<223>  Probe

<400>  123
acaatgagct ccgtgtggcc cc                                                22


<210>  124
<211>  22
<212>  DNA
<213>  Artificial

<220>
<223>  Primer

<400>  124
cgcacgacat ctaggactga gt                                                22


<210>  125

<211>  21
<212>  DNA
<213>  Artificial

<220>
<223>  Primer

<400>  125
cctattcagg cccgacaact t                                                21

<210>  126
<211>  18
<212>  DNA
<213>  Artificial

<220>
<223>  Probe

<400>  126
caccactctg accaaaga                                                    18

<210>  127
<211>  19
<212>  DNA
<213>  Artificial

<220>
<223>  Primer

<400>  127
aagcggtggc agaaaaacc                                                   19

<210>  128
<211>  21
<212>  DNA
<213>  Artificial

<220>
<223>  Primer

<400>  128
gtgctgagcc cttctgaatc a                                                21

<210> 129
<211> 19
<212> DNA
<213> Artificial

<220>
<223> Probe

<400> 129
tggttgaaga ctagcaatg 19


<210> 130
<211> 19
<212> DNA
<213> Artificial

<220>
<223> Primer

<400> 130
ttgtgaatgt cccacccca 19


<210> 131
<211> 23
<212> DNA
<213> Artificial

<220>
<223> Primer

<400> 131
ggtcccatac tggtaggaat cca 23


<210> 132
<211> 25
<212> DNA
<213> Artificial

<220>
<223> Probe

<400> 132
cggtgagaaa gccttgatct cgcct 25

<210>  133
<211>  22
<212>  DNA
<213>  Artificial

<220>
<223>  Primer

<400>  133
acattgagat tgccgcttac ag                                                22


<210>  134
<211>  20
<212>  DNA
<213>  Artificial

<220>
<223>  Primer

<400>  134
accaaagcca atccgacact                                                   20


<210>  135
<211>  15
<212>  DNA
<213>  Artificial

<220>
<223>  Probe

<400>  135
aagctcctgg aaggc                                                        15


<210>  136
<211>  23
<212>  DNA
<213>  Artificial

<220>
<223>  Primer

<400>  136
caatcaggcc tggaaataca ttg                                               23

<210> 137
<211> 19
<212> DNA
<213> Artificial

<220>
<223> Primer

<400> 137
tccgcggctc caagtagtt 19

<210> 138
<211> 21
<212> DNA
<213> Artificial

<220>
<223> Probe

<400> 138
tgacgaatga gattttcact c 21

## Description

### TECHNICAL FIELD

The present invention relates to a method for screening for a regenerative medicine.

### BACKGROUND OF THE INVENTION

Regeneration is a phenomenon in which deficient cells and tissue in a living body are restored by the proliferation and differentiation of stem cells and the like. The constant renewal of cells is called physiological regeneration, for which new cells are supplied as a replacement for cells whose life has been expired as seen in the skin and the gastric and intestinal epithelium; the supplement and recovery of cells and tissue rapidly lost due to injury or disease is called pathological regeneration. The regeneration is an essential phenomenon for the survival of multicellular organisms; however, instinctive regenerative power has limitations in higher animals such as human, and the organ or tissue having received severe or extensive damage therebeyond is not recovered and causes crisis in the life support of the whole individual. For the failure of some organs important for life support such as the kidney, liver and heart, although therapy by organ transplantation has already been established, there was a limit to the number of patients who can benefit from the therapy due to problems with securement of donors, immunocompatibility, etc. Regenerative medicine has recently been a focus of attention as it overcomes such problems in organ transplantation. This is to develop technologies that control the capability inhered in a living body in relation to the generation or regeneration of tissues and effect the tissue reconstruction or organ regeneration using autologous cells or those collected from another person as a material. Technologies that have been already clinically used include bone marrow transplantation carried out for many diseases including leukemia, skin transplantation for bums, and islet transplantation for diabetes. In addition, technologies are also expected to be clinically applied, such as neural stem cell transplantation for Parkinson's disease, bone marrow cell transplantation for myocardial infarction, and Schwann cell transplantation for spinal cord injury. Technologies using proliferation and differentiation factors have already been used in clinical sites, such as wound healing acceleration using fibroblast growth factor (FGF) and anemia therapy using erythropoietin, and the revascularization and the like using hepatocellular growth factor (HGF) or the gene thereof are also expected to be put into practical use as a medicine.

Known techniques associated with the screening of a regenerative medicine are as follows.

WO2006-6722 (Patent Document 1) discloses a method for screening for a gene involved in the regeneration, proliferation or differentiation of cells, comprising the step of quantitatively analyzing the levels of expression of a plurality of genes simultaneously in cells having a regeneration, differentiation or proliferation capability to thereby identify the gene involved in the regeneration, proliferation or differentiation of the cells.

Desbordes et al. have reported an example of widely screening compounds involved in differentiation and self-renewal (screening compounds capable of changing the expression of an undifferentiation marker, Oct4, in hES cells to identify 14 compounds out of 2,880 compounds) (Non-Patent Document 1).

Hahn et al. have reported a method for screening for a therapeutic agent for brain tumor based on HDAC inhibitor and the like (Non-Patent Document 2).

Stegmaier et al. have reported a screening method using Multiplex RT-PCR (a 384-well plate) (Non-Patent Document 3).

### [Citation List]

### [Patent Document]

Patent Document 1: WO2006-6722

### [Non-Patent Documents]

Non-Patent Document 1: Cell Stem Cell, 2 (2008) p.602-612
Non-Patent Document 2: PNAS, 105 (2008) p.9751-9756
Non-Patent Document 3: Nature Genetics 36 (2004) p.257-263

### SUMMARY OF INVENTION

### [Problem to be Solved by the Invention]

Heretofore, the types of cells and organs which can be artificially prepared using stem cells (embryonic stem cells, somatic stem cells, etc.), their precursor cells, and the like have been limited. As for many cells and organs such as pancreatic β-cells, kidney and alimentary tract, the production of clinically applicable cells or tissues has not yet been achieved. In addition, even though certain cells are useful to some extent for regenerative medicine, they do not often reach the level sufficient to completely compensate the lost function. In order to make those cells or tissues which are highly demanded for clinical purposes, but cannot be or are less efficiently prepared available for regenerative medicine, technologies are necessary for more efficiently controlling the differentiation of cells than before. If a new mechanism for regulating cell differentiation that is unraveled during technology development was applied, a new system for screening candidate drugs to promote tissue reconstruction or organ regeneration *in vivo* or *in vitro* could be built up. In particular, if a pharmaceutical product activating stem cells present in a living body and promoting regeneration was developed, patient's burdens such as surgery or use of an immunosuppressant could be eliminated. In addition, if all cells and tissues of human or higher animals were to be mass-produced efficiently, they could be applied to the screening, drug efficacy evaluation, safety studies and the like of candidate drugs in conventional drug discovery processes.

Many issues still remain to be clarified in the differentiation-regulating process in cells. Therefore, there is a limit in the regulation of regeneration or differentiation using known proliferation or differentiation factors, cytokines, inhibitors and activators and the like for signal transduction system. Actually, the types of cells, tissues and organs are extremely limited which could previously be regenerated *in vitro* or *in vivo* using known differentiation-regulating substances. A substance capable of regulating a process of differentiation into a particular cell can be obtained by a screening using stem cells committed to a particular cell lineage; however, the possibility for the substance obtained by such a screening to also have the action on other cells need to be verified in other differentiation systems. In addition, although it is desirable to use stem cells obtained from human tissue to screen for a substance capable of regulating the regeneration or differentiation of human cells, there are constrains in terms of the availability of materials, the mass-preparation of cells for screening, and the like. Thus, stem cells which can be used are limited to mesenchymal stem cells derived from the bone marrow and fat, neural stem cells derived from a fetus, and the like.

As seen above, an endogenous mechanism capable of regulating regeneration or differentiation is present in a living body, and the differentiation/proliferation of cells can be artificially regulated using the mechanism. However, because an actual mechanism of regulating regeneration or differentiation includes a large amount of unresolved aspects, there has been a limitation in a method in which a previously known differentiation/proliferation factor is allowed to act on cells. The mechanism associated with regeneration or differentiation is diverse; thus, in order to find a substance that can be a candidate for a regeneration- or differentiation-regulating agent or a tool for efficiently adjusting regeneration or differentiation, it is necessary to invent a method for widely and quantitatively examining the action.

### [Solution to the Problem]

As a result of intensive studies, the present inventors have found a method for screening for a regenerative medicine, comprised of a process in which: an embryoid body is formed from embryonic stem (ES) cells to induce differentiation in the multi-direction; the embryoid body is dispersed into single cells by trypsin treatment; a compound to be evaluated is then added thereto at the timing of conducting adhesive culturing; and changes in the expression of two or more genes are examined. In addition, it has been found useful to select a gene that is to be used for evaluating changes in expressions of genes with a candidate compound using the following criteria as an indicator: a (known) gene (i) demonstrating an increase or a decrease in an amount during differentiation, (ii) being detectable as significant in its expression even in the absence of the candidate compound (control), and (iii) demonstrating its change in expression in a pilot test using known proliferation/differentiation factors. Since there generally is a common mechanism for regeneration or differentiation among vertebrate animals, it has also been found that findings with respect to pluripotent cells, e.g., mouse ES cells, which can be easily prepared, can also be widely applied to the regulation of regeneration or differentiation of human tissue stem cells; that a common regeneration or differentiation mechanism exists among various stem cells and a substance acting on certain stem cells can also act on other stem cells; and the like. The present inventors have conducted further studies based on these findings, and finally accomplished the present invention.

Thus, the present invention provides the following method for screening (hereinafter sometimes referred to as "the screening method of the present invention").

[1] A method for screening for a substance capable of regulating the regeneration, proliferation or differentiation of a cell or an organ, comprising the following steps (1) to (5):
(1) allowing a cell having a regenerative, proliferative or differentiative capability to form an embryoid body;
(2) treating the embryoid body produced in the step (1) with a digestive enzyme to prepare single cells from the embryoid body;
(3) seeding the cells prepared in the step (2) onto an adhesive plate, and adding a candidate substance to the plate to conduct adhesion culturing of the cells on the plate;
(4) conducting quantitative and simultaneous analysis of the levels of expression of at least two types of genes involved in the regeneration, proliferation or differentiation of cells after the adhesion culturing of the step (3); and
(5) evaluating the influence of the candidate substance on the regeneration, proliferation or differentiation of cells based on the results of the quantitative analysis obtained in the step (4).

[2] The method according to [1] above, wherein the cell in the step (1) is selected from the group consisting of an embryonic stem cell and an iPS (induced pluripotent stem) cell of a human and a warm-blooded animal.

[3] The method according to [1] or [2] above, wherein the cell in the step (1) is cultured for a period of 3 to 6 days to form an embryonic body.

[4] The method according to any of [1] to [3] above, wherein the cell is selected, as a target of the substance capable of regulating the regeneration, proliferation or differentiation, from the group consisting of an embryonic stem cell and an iPS (induced pluripotent stem) cell of a human and a warm-blooded animal, a cell obtained by inducing differentiation of these cells, and a tissue stem cell (a mesenchymal stem cell, a hematopoietic stem cell, a myoblast cell, a neural stem cell, an osteoblast cell, a chondroblast, an angioblastic cell, or a precursor or a stem cell present in a living body) present in a living tissue or an *in vitro* culture.

[5] The method according to any of [1] to [4] above, wherein the substance capable of regulating the regeneration, proliferation or differentiation is a synthetic compound, a natural product, a protein, a peptide, a lipid, an amine, an amino acid, a sugar, a nucleic acid, or an ion.

[6] The method according to any of [1] to [4] above, wherein the substance capable of regulating the regeneration, proliferation or differentiation is selected from the group consisting of an agonist and an antagonist of a receptor, a biosynthetic pathway inhibitor, an inhibitor of protein-protein interaction, an inhibitor and a substrate of an enzyme, a coenzyme, an inhibitor and an activator of signal transduction system, an inhibitor and a modulator of channel, a vitamin, an antioxidant, an inhibitor and a promoter of apoptosis, an antiviral agent, a surfactant, an anti-sense oligonucleotide, siRNA, an antibiotic, a compound synthesized by a combinatorial chemistry method, and synthetic intermediates thereof.

[7] The method according to any of [1] to [6] above, wherein the cell or the organ targeted by the substance capable of regulating the regeneration, proliferation or differentiation is selected from the group consisting of a spleen cell, a neuronal cell, a glial cell, a pancreatic β cell, a bone marrow cell, a mesangial cell, a Langerhans cell, an epidermal cell, an epithelial cell, an endothelial cell, a fibroblast cell, a fibrocyte, a muscle cell, a fat cell, immune cells (a macrophage, a T cell, a B cell, a natural killer cell, a mast cell, a neutrophil, a basophil, an eosinophil, a monocyte, and a megakaryocyte), a synovial cell, a chondrocyte, a bone cell, an osteoblast, an osteoclast, a mammary gland cell, a hepatocyte or interstitial cell, or precursor cells thereof, a stem cell, a blood cell-based cell, brain, regions of the brain (olfactory bulb, amygdaloid nucleus, basal ganglia, hippocampus, thalamus, hypothalamus, subthalamic nucleus, cerebral cortex, medulla oblongata, cerebellum, occipital lobe, frontal lobe, temporal lobe, putamen, caudate nucleus, corpus callosum, and substantia nigra), spinal cord, hypophysis, stomach, pancreas, kidney, liver, gonad, thyroid, gall-bladder, bone marrow, adrenal gland, skin, muscle, lung, gastrointestinal tract (large intestine and small intestine), blood vessel, heart, thymus, spleen, submandibular gland, peripheral blood, a peripheral blood cell, prostate, testicle, testis, ovary, placenta, uterus, bone, joint, and skeletal muscle.

[8] The method according to any of [1] to [7], wherein the substance capable of regulating the regeneration, proliferation or differentiation is a prophylactic and therapeutic agent for a disease selected from the group consisting of central diseases (Alzheimer's disease, Parkinson's disease, and ischemic neuropathy), inflammatory diseases (allergic diseases, asthma, rheumatism, and osteoarthritis), circulatory diseases (heart failure, heart hypertrophy, angina pectoris, and arterial sclerosis), cancers (non-small-cell lung cancer, ovarian cancer, prostate cancer, gastric cancer, bladder cancer, breast cancer, cervical cancer, colon cancer, and rectal cancer), diabetes, immune system diseases (autoimmune disease, atopic dermatitis, allergic diseases, immunodeficiency, asthma, rheumatoid arthritis, psoriasis, arterial sclerosis, diabetes, and Alzheimer's disease), liver/gallbladder diseases (hepatic cirrhosis, hepatitis, hepatic insufficiency, and cholestasis), alimentary diseases (ulcer, enteritis, indigestion, irritable bowel syndrome, ulcerative colitis, diarrhea, and ileus), heat bum, bone fracture, and alopecia.

[9] The method according to any of [1] to [8] above, wherein the digestive enzyme in the step (2) is trypsin.

[10] The method according to any of [1] to [9] above, wherein the adhesive plate in the step (3) is a gelatin-coated plate having a plurality of wells.

[11] The method according to any of [1] to [10] above, wherein the quantitative and simultaneous analysis of the levels of expression of at least two types of genes in the step (4) is performed by Multiplex RT-PCR.

The method according to any of [1] to [11] above, wherein the genes involved in the regeneration, proliferation or differentiation of cells in the step (4) are selected from the group consisting of:
(A) undifferentiation markers, Nanog, Oct3/4, Sox2, Klf4 and Akp2;
(B) a primitive ectoderm marker, Fgf5;
(C) primitive streak markers, Brachyury and Snail 1;
(D) trophectoderm markers, Cdx2 and Bmp4;
(E) neural markers, Tubb3, Nefh, Nestin and p75NTR;
(F) myocardial marker, Actc1;
(G) smooth muscle markers, Acta2 and Con1;
(H) an endothelial cell marker, Tie2;
(I) a mesoderm marker, Flk1;
(J) a marker for mesoderm and endoderm, Cxcr4;
(K) markers for extraembryonic endoderm, Gata4 and Laminin B 1;
(L) skeletal muscle markers, Acta1 and Tpm1;
(M) an osteoblast cell marker, Opn;
(N) a hematopoietic stem cell marker, c-kit; and
(O) a chondrocyte marker, Sox9.

### BRIEF DESCRIPTION OF DRAWINGS

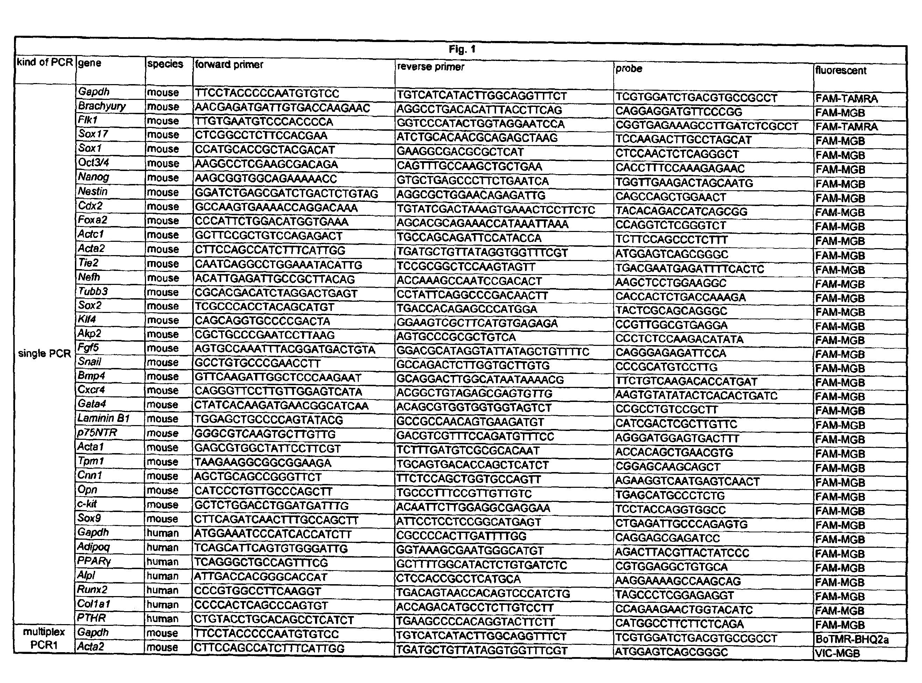

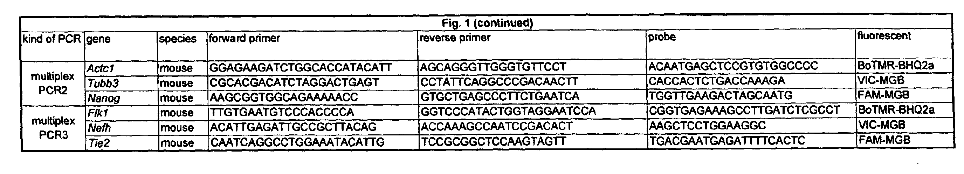

[Figure 1] Figure 1 is a table showing the sequences of the primers and probe used for the measurement of the level of expression of each gene.

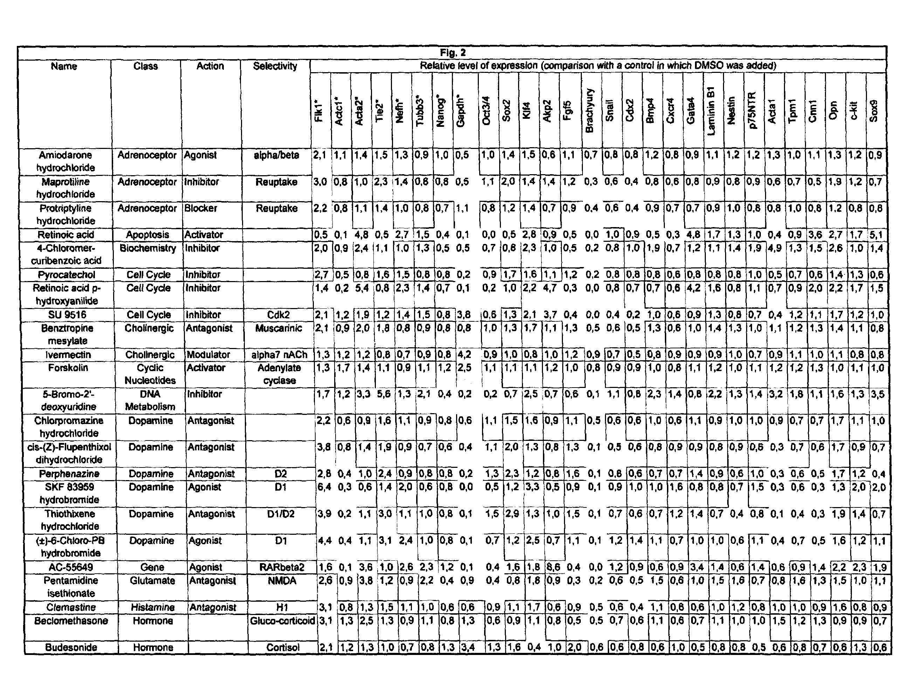

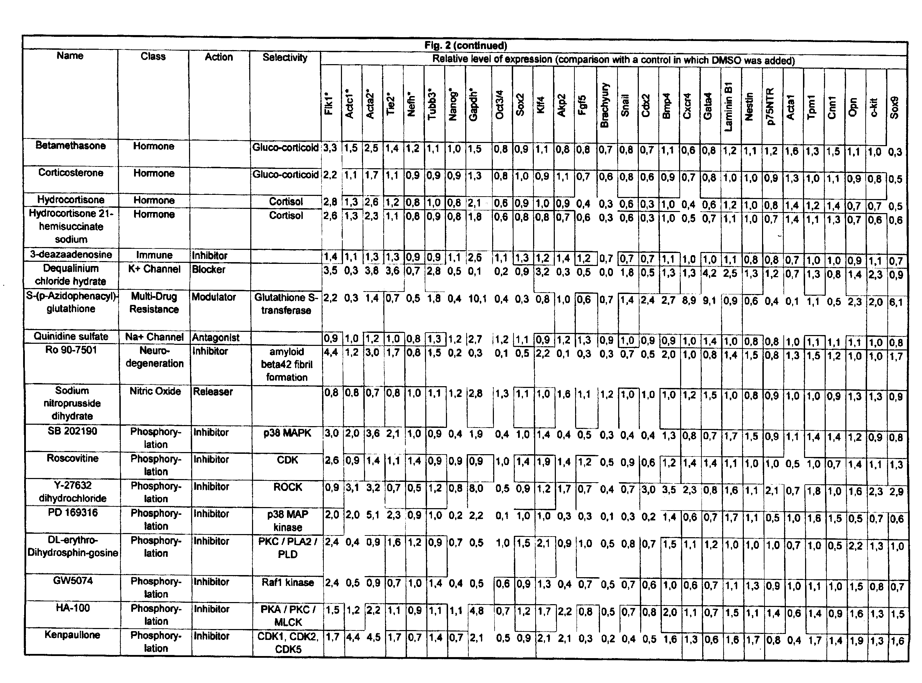

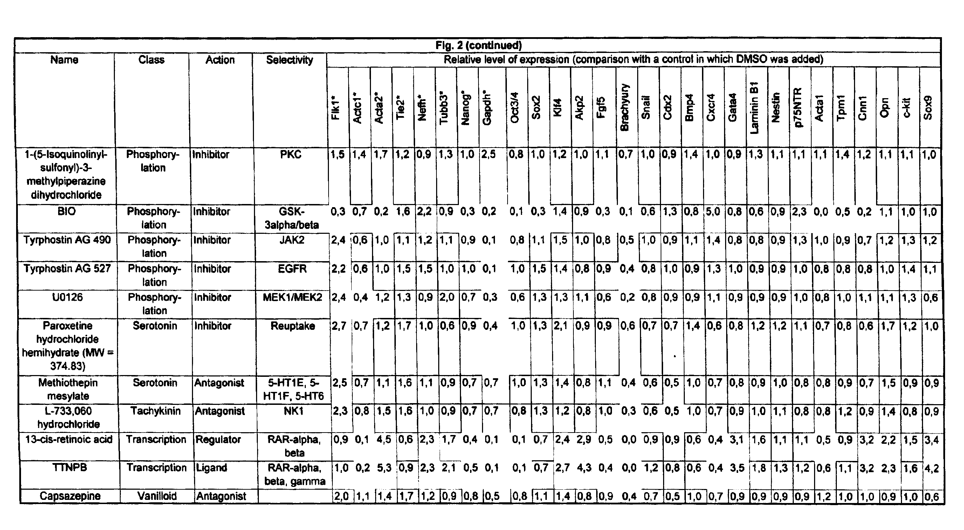

[Figure 2] Figure 2 is a table showing 52 compounds that showed an increase by 2 times or more in the level of expression of at least one gene as compared with a control in which DMSO was added by a screening method of the present invention using LOPAC¹²⁸⁰ (Sigma). The relative level of expression of each gene for which each compound was added is shown. The genes marked with * indicate the 8 types of genes measured in the screening. The case of an increase by 2 times or more that of a control in which DMSO was added are colored in blue and the case of a decrease by 0.5 time or less is colored in red.

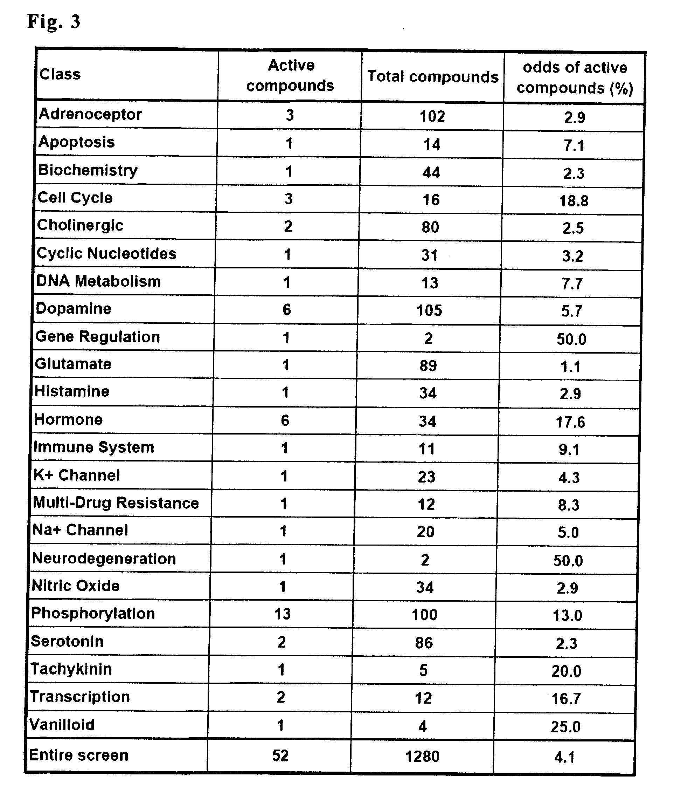

[Figure 3] Figure 3 is a table showing 52 compounds which were selected by screening and classified based on their pharmacological actions.

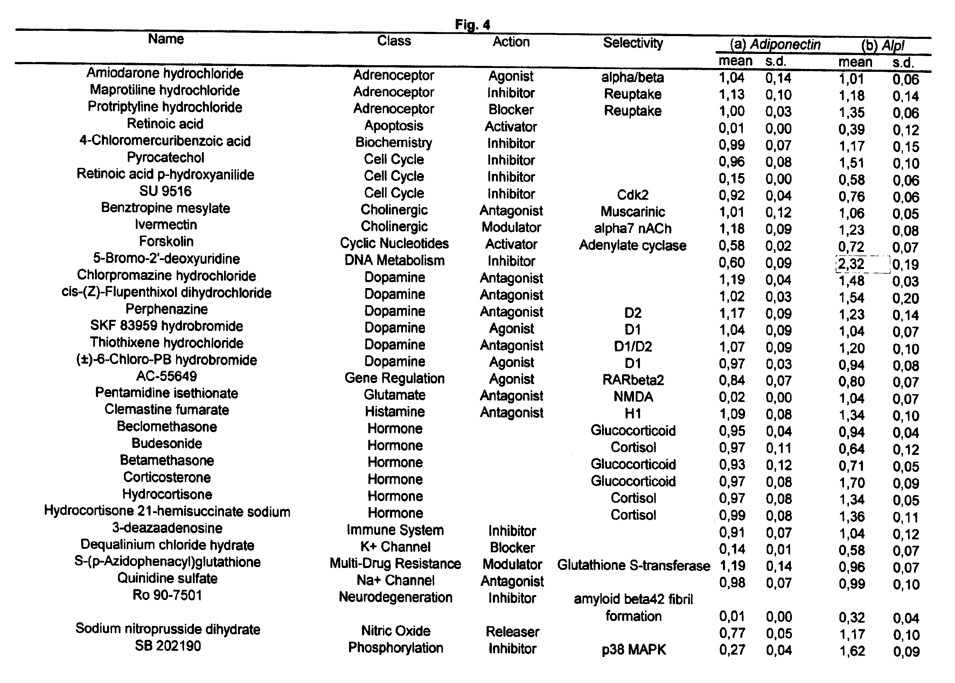

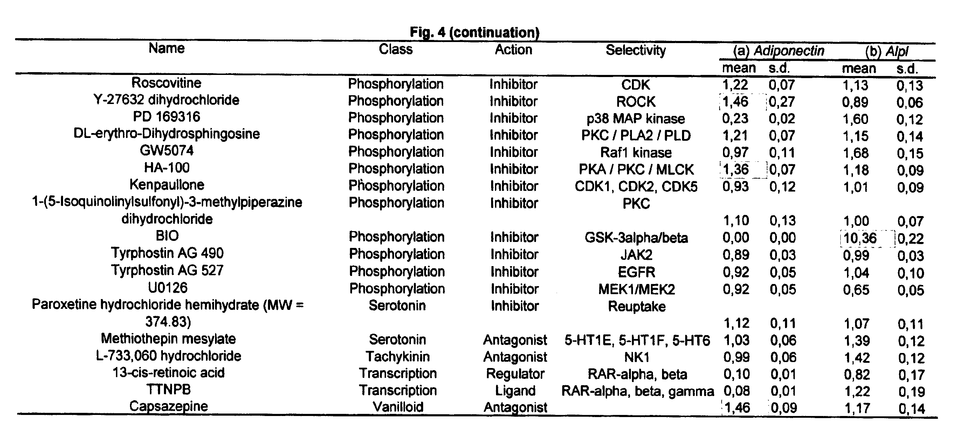

[Figure 4] Figure 4 (a) shows the level of expression of adiponectin for which adipose differentiation of a human mesenchymal stem cell was induced simultaneously with an addition of each of the 52 compounds shown in Figure 2, followed by adhesion culturing for 5 days. The values in the table indicate the relative levels of expression to that of a control in which DMSO was added. Shown in blue is one for which an increase by 1.3 times or more that of a control, in which DMSO was added, was observed, and shown in red is one for which a decrease by 0.5 time or less was observed. Figure 4 (b) shows the level of expression of Alpl for which bone differentiation of a human mesenchymal stem cell was induced simultaneously with an addition of each of the 52 compounds shown in Figure 2 followed by adhesion culturing for 8 days. The values in the table indicate the relative levels of expression to that of a control in which DMSO was added. Shown in blue is the case for which an increase by 2 times or more as compared with a control, in which DMSO was added, was observed, and shown in red is the case for which a decrease by 0.5 time or less was observed.

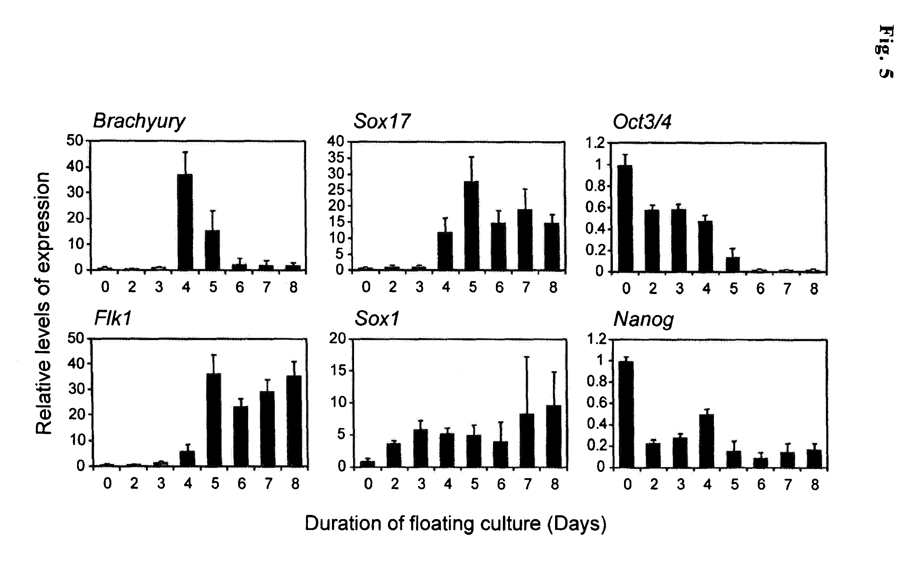

[Figure 5] Figure 5 is a series of graphs showing the levels of expression of initial differentiation markers for which embryoid bodies were formed in a floating culture using a 96-well spheroid-plate. The values in the figure indicate the relative levels of expression to the level of expression in undifferentiated ES cells at day 0 of culture.

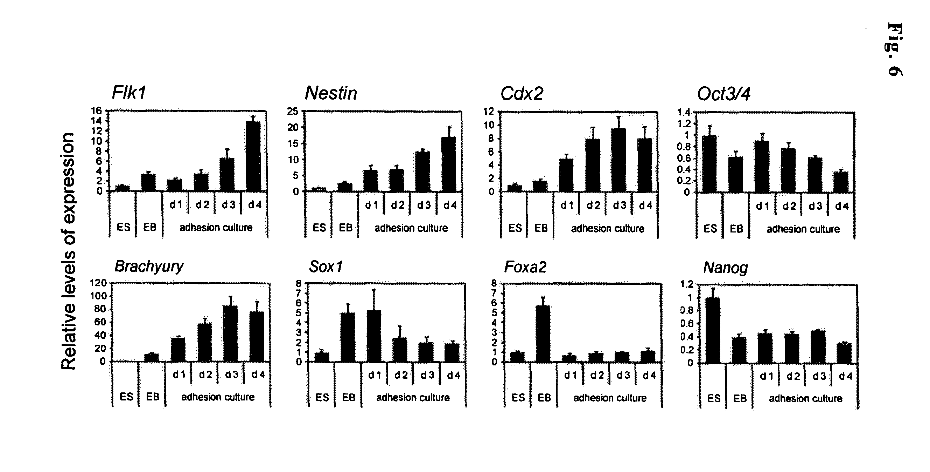

[Figure 6] Figure 6 is a series of graphs showing the levels of expression of initial differentiation markers in undifferentiated ES cells (ES), embryoid bodies (at day 4 of floating culturing, EB) and in an adhesion culture at day 1 (d1) to 4 (d4). The values in the figure indicate the relative levels of expression to the level of expression in undifferentiated ES cells at day 0 of culture.

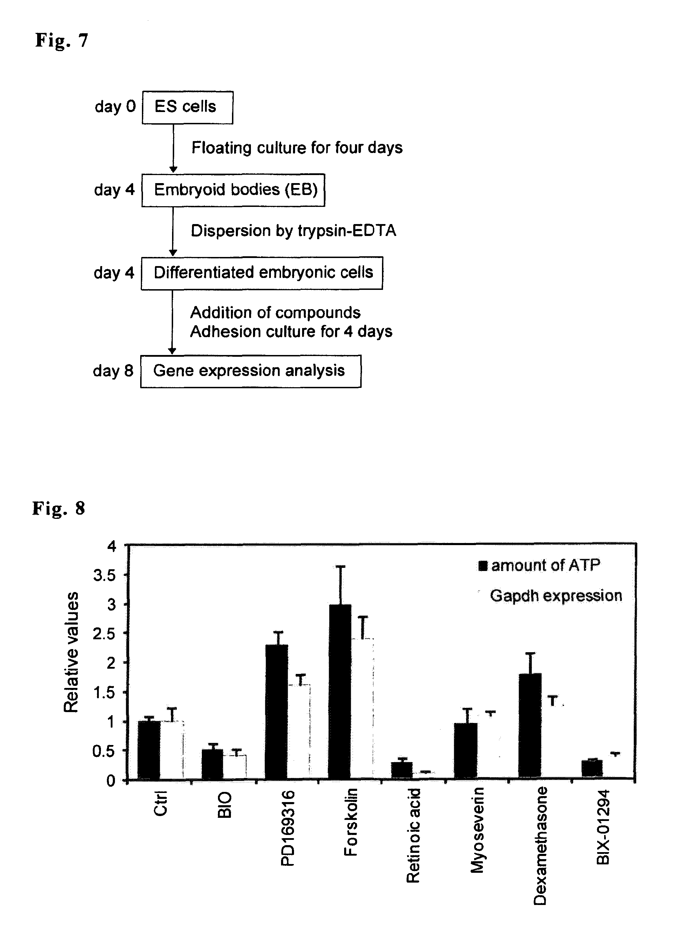

[Figure 7] Figure 7 is a schematic diagram showing the screening method of the present invention. The screening method of the present invention is conducted in a following procedure: allowing formation of embryoid bodies in a floating culture for 4 days; preparing single cells with a trypsin-EDTA solution; adding a compound and performing adhesion culturing for further 4 days, and examining the level of expression of each differentiation marker.
[Figure 8] Figure 8 is a graph showing the results of measurements of the level of GAPDH expression and the amount of ATP for which each compound in the graph was added according to the method shown in Figure 7 before adhesion culturing for 4 days. The values in the graph indicate the relative values to that of a control in which DMSO was added.

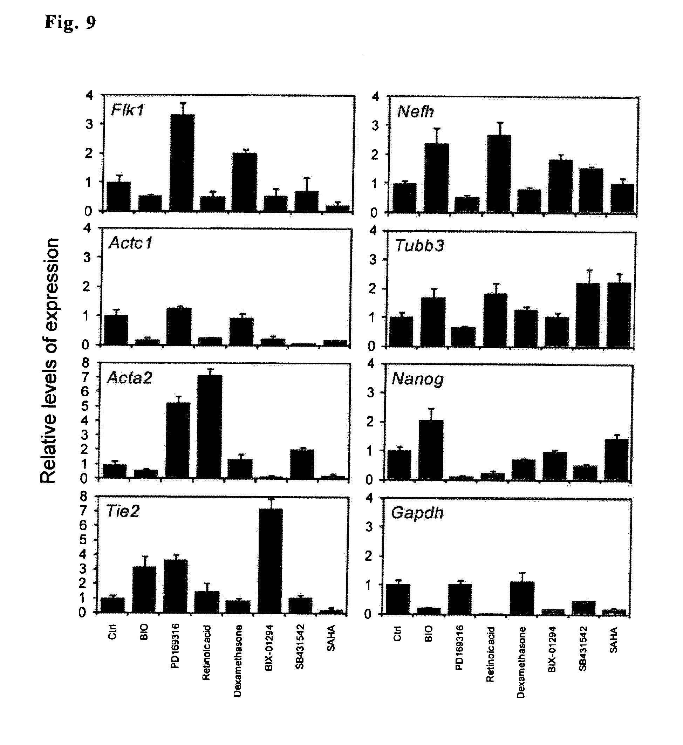

[Figure 9] Figure 9 is a series of graphs showing the levels of expression of various genes for which each compound in the graph was added according to the method shown in Figure 7 before adhesion culturing for 4 days. The values in the graph indicate the relative levels of expression to that of a control in which DMSO was added.

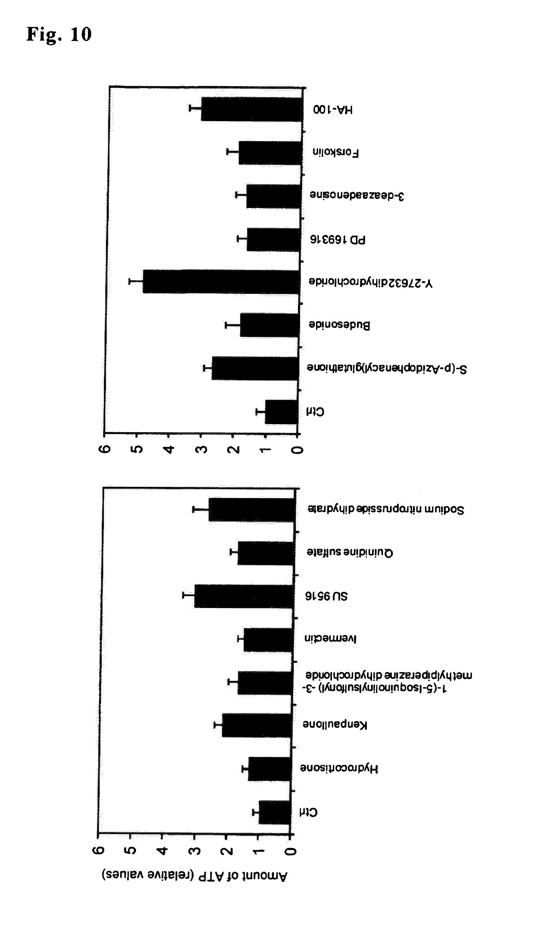

[Figure 10] Figure 10 is a pair of graphs showing the results of measurements of the amount of ATP for which each compound in the graph was added according to the method shown in Figure 7 before adhesion culturing for 4 days. The values in the graph indicate the relative values to that of a control in which DMSO was added.

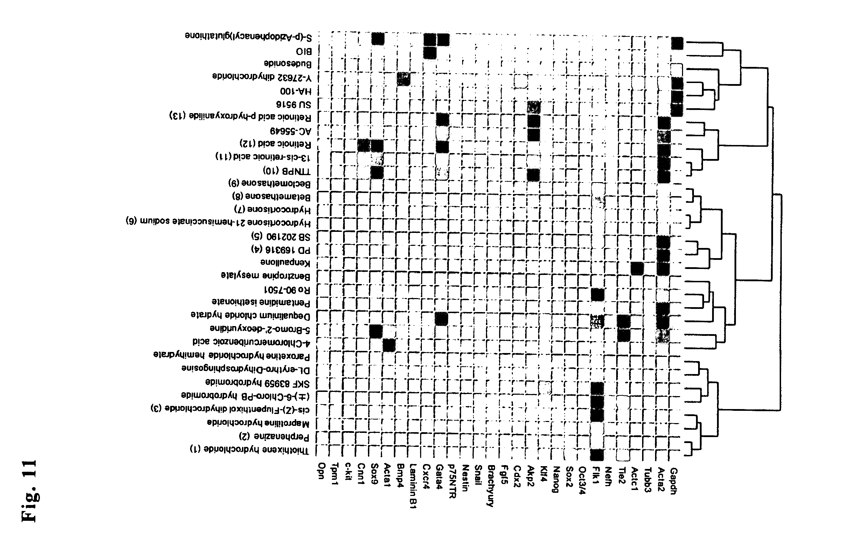

[Figure 11] Figure 11 is a diagram showing the result of clustering, based on correlation distance, of the expression profile data of 28 types of genes for 32 of the compounds selected by screening. The compounds 1 to 3 are dopamine-receptor antagonists; the compounds 4 and 5 are p38 MAPK inhibitors; the compounds 6 to 9 are corticosteroids; and the compounds 10 to 13 are retinoic acid receptor agonists.

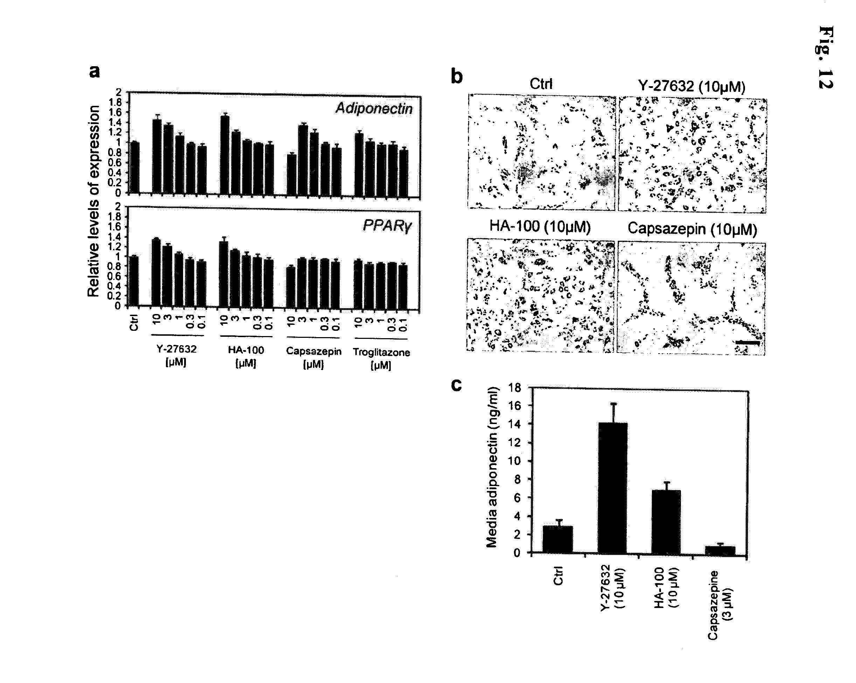

[Figure 12] Figure 12(a) is a pair of graphs showing the levels of expression of adiponectin and PPARγ for which adipose differentiation of a human mesenchymal stem cell was induced and simultaneously each of the compounds shown in the figure was added before adhesion culturing for 5 days. The values in the graph indicate the relative levels of expression to that of a control in which DMSO was added. Figure 12(b) is a drawing showing the results obtained from inducing adipose differentiation of a human mesenchymal stem cell and simultaneously each of the compounds shown in the figure was added to adhesion culturing for 5 days, followed by staining a fat droplet with an oil red O solution. Figure 12(c) is a graph showing the results obtained from inducing adipose differentiation of a human mesenchymal stem cell and simultaneously each of the compounds shown in the figure was added to adhesion culturing for 8 days, followed by a measurement of the amount of adiponectin contained in the culture solution.

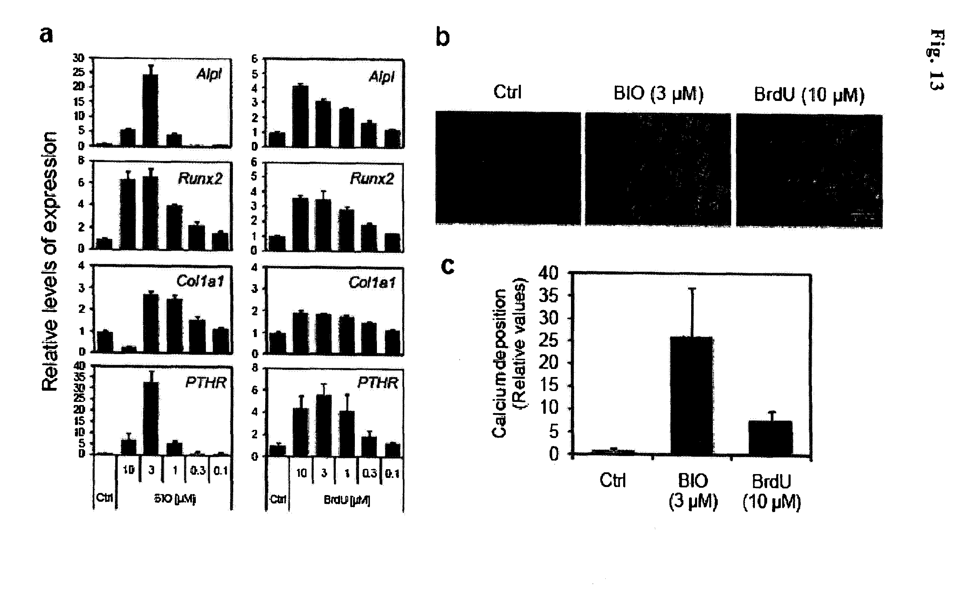

[Figure 13] Figure 13(a) is a series of graphs showing the levels of expression of Alpl, Runx2, Colla1 and PTHR for which bone differentiation from a human mesenchymal stem cell was induced and simultaneously each of the compounds shown in the figure was added to adhesion culturing for 8 days. The values in the graph indicate the relative levels of expression to that of a control in which DMSO was added. Figure 13(b) is a drawing showing the results obtained from inducing bone differentiation of a human mesenchymal stem cell and simultaneously adding each of the compounds shown in the figure to adhesion culturing for 8 days, followed by staining with alkaline phosphatase. Figure 13(c) is a graph showing the results of inducing bone differentiation from a human mesenchymal stem cell and simultaneously each of the compounds shown in the figure was added to adhesion culturing for 15 days, followed by a measurement of the amount of calcium accumulated in the cell. The values in the graph indicate the relative values to that of a control in which DMSO was added.

### EMBODIMENTS TO CARRY OUT THE INVENTION

### 1. Method for screening a substance capable of regulating regeneration, proliferation or differentiation of a cell or an organ

According to one embodiment of the present invention, a method for screening for a substance capable of regulating the regeneration, proliferation or differentiation of a cell or an organ (hereinafter sometimes referred to as "the screening method of the present invention") is provided. This method typically comprises the steps of:
(1) allowing a cell having a regenerative, proliferative or differentiative capability to form an embryoid body;
(2) treating the embryoid body produced in the step (1) with a digestive enzyme to prepare single cells from the embryoid body;
(3) seeding the cells prepared in the step (2) onto an adhesive plate, and adding a candidate substance to the plate to perform adhesion culturing of the cells on the plate;
(4) conducting quantitative and simultaneous analysis of the levels of expression of at least two types of genes involved in the regeneration, proliferation or differentiation of cells after the adhesion culturing of the step (3); and
(5) evaluating the influence of the candidate substance on the regeneration, proliferation or differentiation of cells based on the results of the quantitative analysis obtained in the step (4).

According to the screening method of the present invention, in the step (1), an embryoid body (EB) is first formed from a cell having a regenerative, proliferative or differentiative capability (for example, ES cell) to induce differentiation in the multi-direction.

As used herein, the term "embryoid body" has a meaning as generally used in the art and refers to a cluster of cells comprised of stem cells or precursor cells of various tissues in an embryonic form, which are formed when pluripotent stem cells such as ES cells and iPS cells are differentiated in floating culture.

According to the screening method of the present invention, preferred examples of the cell having a regenerative, proliferative or differentiative capability used for forming an embryoid body in the step (1) include embryonic stem cells (ES cells) and iPS (induced pluripotent stem) cells of human and other warm-blooded animals (e.g., mouse). To form an embryoid body, ES cells of a mouse or the like are typically suspended in a medium containing serum or a serum replacement and subjected to floating culturing under conditions of 37°C and 5% CO₂ for 1 to 10 days. Particularly preferred examples of the cell having a regenerative, proliferative or differentiative capability used for forming an embryoid body in the step (1) include embryonic stem cells (ES cells) of human and other warm-blooded animals (e.g., mouse).

Depending on the form of cells in cell culture, a method for culturing cells can be classified into adhesion culturing and floating culturing. The adhesion culturing is a method which involves attaching cultured cells to a culture vessel for proliferation; the floating culturing is a method which involves proliferation of cultured cells in a floating state in a medium.

According to the screening method of the present invention, the cell culturing performed to form an embryoid body is typically carried out in a floating culture. The culturing period is not particularly limited as long as it allows the formation of the embryoid body; however, the period is typically 1 to 6 days. The culturing period is preferably 3 to 6 days. The culturing period is more preferably 4 days.

For example, a non-adhesive multi-well plate can be utilized for forming homogeneous embryoid bodies to obtain stable results.

Next, in the step (2), a digestive enzyme is added to the embryoid body obtained in the step (1) to separate cells into single cells from the embryoid body. The digestive enzyme which can be used includes trypsin, collagenase, papain, dispase, Accutase (trade name), and the like. Among others, trypsin is preferable; it is typically used in a form such as a trypsin-EDTA solution (e.g., 0.25% trypsin-1 mM EDTA) in which EDTA is added to chelate Ca²⁺ and Mg²⁺ as inhibitors of the action of the digestive enzyme.

A homogenous cell population derived from the embryoid body can be prepared in the steps (1) and (2) to provide stable results in the screening.

In the step (3), the cells obtained in the step (2) are seeded onto an adhesive plate and a candidate substance is added to the plate for adhesion culturing. As used herein, the "adhesive plate " refers to a plate which can be used for adhesion culturing, and means a plate whose surface is coated with a cell adhesion promoter, for example, fibronectin, type I or IV collagen, laminin, vitronectin, gelatin, Matrigel (trade name), poly-lysine, or poly-ornithine so that the cells can be attached to the plate, spread, and proliferate. Preferred examples of the adhesive plate used for the screening method of the present invention include a gelatin-coated plate having a plurality of wells (e.g., a 96-well plate).

As used herein, the "candidate substance" refers to a candidate for a substance capable of regulating the regeneration, proliferation or differentiation of a cell or an organ, that is, a substance to be screened for by the screening method of the present invention, and includes a synthetic compound, a natural product, a protein, a peptide, a lipid, an amine, an amino acid, a sugar, a nucleic acid, and an ion. The "candidate substance" also includes an agonist and an antagonist of a receptor, a biosynthetic pathway inhibitor, an inhibitor of protein-protein interaction, an inhibitor and a substrate of an enzyme, a coenzyme, an inhibitor and an activator of signal transduction system, an inhibitor and a modulator of channel, a vitamin, an antioxidant, an inhibitor and a promoter of apoptosis, a surfactant, an anti-sense oligonucleotide, siRNA, an antibiotic, an antiviral agent, a compound synthesized by a combinatorial chemistry method, and also includes synthetic intermediates thereof.

The screening method of the present invention is clearly distinguished from a conventional screening system which uses ES cells in the following aspect: In a conventional screening system, evaluation of a compound (a candidate substance) is carried out in a monolayer culture in an undifferentiated state without a step of embryoid body formation while in the present invention, evaluation of the compound (the candidate substance) is performed after digesting and dispersing an embryoid body comprised of cells differentiated in the multi-directions with a digestive enzyme such as trypsin.

In the step (4), the levels of expression of at least two types of genes involved in the regeneration, proliferation or differentiation of cells are analyzed quantitatively and simultaneously after the adhesion culturing of the step (3).

As used herein, the "genes involved in the regeneration, proliferation or differentiation of cells" refer to genes already known to play certain roles in the regeneration, proliferation or differentiation of cells, or genes known to be greatly changed in expression during the regeneration, proliferation or differentiation of cells.

Particularly, the "genes involved in the regeneration, proliferation or differentiation of cells" used for the screening method of the present invention are preferably genes that (i) increase or decrease in expression during differentiation, (ii) can be detected for expression with a significant value in the absence of the compound (control), and (iii) have been demonstrated to change in its expression in a pilot test using a known proliferation/differentiation factor.

Preferred examples of the genes involved in the regeneration, proliferation or differentiation of cells usable in the screening method of the present invention include:
(A) undifferentiation markers, Nanog, Oct3/4, Sox2, Klf4 and Akp2;
(B) a primitive ectoderm marker, Fgf5;
(C) primitive streak markers, Brachyury and Snail1;
(D) trophectoderm markers, Cdx2 and Bmp4;
(E) neural markers, Tubb3, Nefh, Nestin and p75NTR;
(F) a myocardial marker, Actc1;
(G) smooth muscle markers, Acta2 and Cnn1;
(H) an endothelial cell marker, Tie2;
(I) a mesoderm marker, Flk1;
(J) a marker for mesoderm and endoderm, Cxcr4;
(K) markers for extraembryonic endoderm, Gata4 and Laminin B1;
(L) skeletal muscle markers, Acta1 and Tpm1;
(M) an osteoblast cell marker, Opn;
(N) a hematopoietic stem cell marker, c-kit; and
(O) a chondrocyte marker, Sox9.

As used herein, "conducting quantitative and simultaneous analysis of the levels of expression of at least two types of genes" means that when doing the quantitative analysis of the levels of expression of at least two types of genes, at least two types of genes are subjected to the quantitative analysis not sequentially one by one but together in one go. Specifically, for example, the levels of expression of at least two types of genes can be measured at the same time using Multiplex RT-PCR or the like which can measure the levels of expression of a plurality of genes at the same time. Although Multiplex RT-PCR is an existing technique, it is rarely used for high-throughput screening because it requires the intricate setting of the measurement system. For the screening method of the present invention, it is preferable to use Multiplex RT-PCR to increase the throughput of the system in the gene expression analysis. According to the screening method of the present invention, a group of a wide variety of compounds can be obtained which are involved in differentiation; the group of candidate compounds obtained can be used for the induction of differentiation of other adult stem cells or embryonic stem cells.

In conducting quantitative and simultaneous analysis of the levels of expression of at least two types of genes, the measurement is preferably carried out using an indicator reflecting the number of living cells as an internal control. The level of expression of each gene can be corrected with the internal control to eliminate the influence of a compound on an increase or decrease in the number of cells. Examples of the indicator reflecting the number of living cells include the level of expression of Gapdh, the content of ATP, the content of protein, and the content of DNA. The indicator reflecting the number of living cells is preferably the level of expression of Gapdh. The control for observing a change in the level of expression of a gene uses the same solvent as that used for dissolving the compound, such as DMSO, DMF, methanol, ethanol, saline, or a buffer solution. A substance capable of changing (decreasing or increasing) the level of expression of a gene by a factor of 1.2 or more, preferably 1.4 or more, more preferably 1.6 or more, still more preferably 1.8 or more, most preferably 2 or more, compared to the control that was added for observing an change in the level of expression of the gene can be selected as the substance capable of regulating the regeneration, proliferation or differentiation of a cell or an organ.

A desired cell can be efficiently differentiated by an action, on a stem cell, of the substance capable of regulating the regeneration, proliferation or differentiation of a cell or an organ found by the screening method of the present invention, or any combinations of the substances.

### 2. Utility of compounds obtained by the screening method of the present invention

The compound obtained by the screening method of the present invention can be used to act on a cell or an organ to regulate the regeneration, proliferation or differentiation of the cell or the organ. Examples of such a target cell include an embryonic stem cell and an iPS (induced pluripotent stem) cell of a human and a warm-blooded animal, cells obtained by inducing differentiation of these cells, a tissue stem cell (a mesenchymal stem cell present in bone marrow, fat and the like, a hematopoietic stem cell, a myoblast cell, a neural stem cell, an osteoblast cell, a chondroblast, an angioblastic cell, and a precursor or a stem cell present in a living body) present in a living tissue or an *in vitro* culture.

Examples of the cell or the organ regenerated, proliferated, or differentiated by the action of a compound obtained by the screening method of the present invention include a spleen cell, a neuronal cell, a glial cell, a pancreatic β cell, a bone marrow cell, a mesangial cell, a Langerhans cell, an epidermal cell, an epithelial cell, an endothelial cell, a fibroblast cell, a fibrocyte, a muscle cell, a fat cell, immune cells (e.g., a macrophage, a T cell, a B cell, a natural killer cell, a mast cell, a neutrophil, a basophil, an eosinophil, a monocyte, and a megakaryocyte), a synovial cell, a chondrocyte, a bone cell, an osteoblast, an osteoclast, a mammary gland cell, a hepatocyte or interstitial cell, or precursor cells thereof, a stem cell and a hemocyte-related cell, or any tissues in which these cells are present, for example, brain, regions of the brain (e.g., olfactory bulb, amygdaloid nucleus, basal ganglia, hippocampus, thalamus, hypothalamus, subthalamic nucleus, cerebral cortex, medulla oblongata, cerebellum, occipital lobe, frontal lobe, temporal lobe, putamen, caudate nucleus, corpus callosum, and substantia nigra), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, a peripheral blood cell, prostate, testicle, testis, ovary, placenta, uterus, bone, joint, and skeletal muscle.

Preferred examples of the cell or the organ regenerated, proliferated, or differentiated by an action of a compound obtained by the screening method of the present invention include a bone cell and a fat cell.

The compound obtained by the screening method of the present invention, that is, the substance capable of regulating the regeneration, proliferation or differentiation of a cell or an organ, can be used for the treatment of a disease. Examples of the target disease include central diseases (e.g., Alzheimer's disease, Parkinson's disease, and ischemic neuropathy), inflammatory diseases (e.g., allergic diseases, asthma, rheumatism, and osteoarthritis), circulatory diseases (e.g., heart failure, heart hypertrophy, angina pectoris, and arterial sclerosis), cancers (e.g., non-small-cell lung cancer, ovarian cancer, prostate cancer, gastric cancer, bladder cancer, breast cancer, cervical cancer, colon cancer, and rectal cancer), diabetes, immune system diseases (e.g., autoimmune disease, atopic dermatitis, allergic diseases, immunodeficiency, asthma, rheumatoid arthritis, psoriasis, arterial sclerosis, diabetes, and Alzheimer's disease), liver/gallbladder diseases (e.g., hepatic cirrhosis, hepatitis, hepatic insufficiency, and cholestasis, and stone formation), alimentary diseases (e.g., ulcer, enteritis, indigestion, irritable bowel syndrome, ulcerative colitis, diarrhea, and ileus), heat burn, bone fracture, and alopecia.

The compound obtained by the screening method of the present invention is less toxic and can be orally or parenterally administered to mammals (e.g., human), if necessary, after being formulated according to a method known per se. Here, the dosage and administration frequency of the compound may be properly selected depending on a subject to be administered, a target disease, and the like.

The relation between the SEQ ID NOS. shown in the Sequence Listing of this application and sequences is as follows.
[SEQ ID NO: 1]
   It indicates the base sequence of a primer used in Example 1.
[SEQ ID NO: 2]
   It indicates the base sequence of a primer used in Example 1.
[SEQ ID NO: 3]
   It indicates the base sequence of a probe used in Example 1.
[SEQ ID NO: 4]
   It indicates the base sequence of a primer used in Example 1.
[SEQ ID NO: 5]
   It indicates the base sequence of a primer used in Example 1.
[SEQ ID NO: 6]
   It indicates the base sequence of a probe used in Example 1.
[SEQ ID NO: 7]
   It indicates the base sequence of a primer used in Example 1.
[SEQ ID NO: 8]
   It indicates the base sequence of a primer used in Example 1.
[SEQ ID NO: 9]
   It indicates the base sequence of a probe used in Example 1.
[SEQ ID NO: 10]
   It indicates the base sequence of a primer used in Example 1.
[SEQ ID NO: 11]
   It indicates the base sequence of a primer used in Example 1.
[SEQ ID NO: 12]
   It indicates the base sequence of a probe used in Example 1.
[SEQ ID NO: 13]
   It indicates the base sequence of a primer used in Example 1.
[SEQ ID NO: 14]
   It indicates the base sequence of a primer used in Example 1.
[SEQ ID NO: 15]
   It indicates the base sequence of a probe used in Example 1.
[SEQ ID NO: 16]
   It indicates the base sequence of a primer used in Example 1.
[SEQ ID NO: 17]
   It indicates the base sequence of a primer used in Example 1.
[SEQ ID NO: 18]
   It indicates the base sequence of a probe used in Example 1.
[SEQ ID NO: 19]
   It indicates the base sequence of a primer used in Example 1.
[SEQ ID NO: 20]
   It indicates the base sequence of a primer used in Example 1.
[SEQ ID NO: 21]
   It indicates the base sequence of a probe used in Example 1.
[SEQ ID NO: 22]
   It indicates the base sequence of a primer used in Example 5.
[SEQ ID NO: 23]
   It indicates the base sequence of a primer used in Example 5.
[SEQ ID NO: 24]
   It indicates the base sequence of a probe used in Example 5.
[SEQ ID NO: 25]
   It indicates the base sequence of a primer used in Example 5.
[SEQ ID NO: 26]
   It indicates the base sequence of a primer used in Example 5.
[SEQ ID NO: 27]
   It indicates the base sequence of a probe used in Example 5.
[SEQ ID NO: 28]
   It indicates the base sequence of a primer used in Example 2.
[SEQ ID NO: 29]
   It indicates the base sequence of a primer used in Example 2.
[SEQ ID NO: 30]
   It indicates the base sequence of a probe used in Example 2.
[SEQ ID NO: 31]
   It indicates the base sequence of a primer used in Example 5.
[SEQ ID NO: 32]
   It indicates the base sequence of a primer used in Example 5.
[SEQ ID NO: 33]
   It indicates the base sequence of a probe used in Example 5.
[SEQ ID NO: 34]
   It indicates the base sequence of a primer used in Example 5.
[SEQ ID NO: 35]
   It indicates the base sequence of a primer used in Example 5.
[SEQ ID NO: 36]
   It indicates the base sequence of a probe used in Example 5.
[SEQ ID NO: 37]
   It indicates the base sequence of a primer used in Example 5.
[SEQ ID NO: 38]
   It indicates the base sequence of a primer used in Example 5.
[SEQ ID NO: 39]
   It indicates the base sequence of a probe used in Example 5.
[SEQ ID NO: 40]
   It indicates the base sequence of a primer used in Example 5.
[SEQ ID NO: 41]
   It indicates the base sequence of a primer used in Example 5.
[SEQ ID NO: 42]
   It indicates the base sequence of a probe used in Example 5.
[SEQ ID NO: 43]
   It indicates the base sequence of a primer used in Example 5.
[SEQ ID NO: 44]
   It indicates the base sequence of a primer used in Example 5.
[SEQ ID NO: 45]
   It indicates the base sequence of a probe used in Example 5.
[SEQ ID NO: 46]
   It indicates the base sequence of a primer used in Example 5.
[SEQ ID NO: 47]
   It indicates the base sequence of a primer used in Example 5.
[SEQ ID NO: 48]
   It indicates the base sequence of a probe used in Example 5.
[SEQ ID NO: 49]
   It indicates the base sequence of a primer used in Example 5.
[SEQ ID NO: 50]
   It indicates the base sequence of a primer used in Example 5.
[SEQ ID NO: 51]
   It indicates the base sequence of a probe used in Example 5.
[SEQ ID NO: 52]
   It indicates the base sequence of a primer used in Example 5.
[SEQ ID NO: 53]
   It indicates the base sequence of a primer used in Example 5.
[SEQ ID NO: 54]
   It indicates the base sequence of a probe used in Example 5.
[SEQ ID NO: 55]
   It indicates the base sequence of a primer used in Example 5.
[SEQ ID NO: 56]
   It indicates the base sequence of a primer used in Example 5.
[SEQ ID NO: 57]
   It indicates the base sequence of a probe used in Example 5.
[SEQ ID NO: 58]
   It indicates the base sequence of a primer used in Example 5.
[SEQ ID NO: 59]
   It indicates the base sequence of a primer used in Example 5.
[SEQ ID NO: 60]
   It indicates the base sequence of a probe used in Example 5.
[SEQ ID NO: 61]
   It indicates the base sequence of a primer used in Example 5.
[SEQ ID NO: 62]
   It indicates the base sequence of a primer used in Example 5.
[SEQ ID NO: 63]
   It indicates the base sequence of a probe used in Example 5.
[SEQ ID NO: 64]
   It indicates the base sequence of a primer used in Example 5.
[SEQ ID NO: 65]
   It indicates the base sequence of a primer used in Example 5.
[SEQ ID NO: 66]
   It indicates the base sequence of a probe used in Example 5.
[SEQ ID NO: 67]
   It indicates the base sequence of a primer used in Example 5.
[SEQ ID NO: 68]
   It indicates the base sequence of a primer used in Example 5.
[SEQ ID NO: 69]
   It indicates the base sequence of a probe used in Example 5.
[SEQ ID NO: 70]
   It indicates the base sequence of a primer used in Example 5.
[SEQ ID NO: 71]
   It indicates the base sequence of a primer used in Example 5.
[SEQ ID NO: 72]
   It indicates the base sequence of a probe used in Example 5.
[SEQ ID NO: 73]
   It indicates the base sequence of a primer used in Example 5.
[SEQ ID NO: 74]
   It indicates the base sequence of a primer used in Example 5.
[SEQ ID NO: 75]
   It indicates the base sequence of a probe used in Example 5.
[SEQ ID NO: 76]
   It indicates the base sequence of a primer used in Example 5.
[SEQ ID NO: 77]
   It indicates the base sequence of a primer used in Example 5.
[SEQ ID NO: 78]
   It indicates the base sequence of a probe used in Example 5.
[SEQ ID NO: 79]
   It indicates the base sequence of a primer used in Example 5.
[SEQ ID NO: 80]
   It indicates the base sequence of a primer used in Example 5.
[SEQ ID NO: 81]
   It indicates the base sequence of a probe used in Example 5.
[SEQ ID NO: 82]
   It indicates the base sequence of a primer used in Example 5.
[SEQ ID NO: 83]
   It indicates the base sequence of a primer used in Example 5.
[SEQ ID NO: 84]
   It indicates the base sequence of a probe used in Example 5.
[SEQ ID NO: 85]
   It indicates the base sequence of a primer used in Example 5.
[SEQ ID NO: 86]
   It indicates the base sequence of a primer used in Example 5.
[SEQ ID NO: 87]
   It indicates the base sequence of a probe used in Example 5.
[SEQ ID NO: 88]
   It indicates the base sequence of a primer used in Example 5.
[SEQ ID NO: 89]
   It indicates the base sequence of a primer used in Example 5.
[SEQ ID NO: 90]
   It indicates the base sequence of a probe used in Example 5.
[SEQ ID NO: 91]
   It indicates the base sequence of a primer used in Example 5.
[SEQ ID NO: 92]
   It indicates the base sequence of a primer used in Example 5.
[SEQ ID NO: 93]
   It indicates the base sequence of a probe used in Example 5.
[SEQ ID NO: 94]
   It indicates the base sequence of a primer used in Example 6.
[SEQ ID NO: 95]
   It indicates the base sequence of a primer used in Example 6.
[SEQ ID NO: 96]
   It indicates the base sequence of a probe used in Example 6.
[SEQ ID NO: 97]
   It indicates the base sequence of a primer used in Example 6.
[SEQ ID NO: 98]
   It indicates the base sequence of a primer used in Example 6.
[SEQ ID NO: 99]
   It indicates the base sequence of a probe used in Example 6.
[SEQ ID NO: 100]
   It indicates the base sequence of a primer used in Example 6.
[SEQ ID NO: 101]
   It indicates the base sequence of a primer used in Example 6.
[SEQ ID NO: 102]
   It indicates the base sequence of a probe used in Example 6.
[SEQ ID NO: 103]
   It indicates the base sequence of a primer used in Example 7.
[SEQ ID NO: 104]
   It indicates the base sequence of a primer used in Example 7.
[SEQ ID NO: 105]
   It indicates the base sequence of a probe used in Example 7.
[SEQ ID NO: 106]
   It indicates the base sequence of a primer used in Example 7.
[SEQ ID NO: 107]
   It indicates the base sequence of a primer used in Example 7.
[SEQ ID NO: 108]
   It indicates the base sequence of a probe used in Example 7.
[SEQ ID NO: 109]
   It indicates the base sequence of a primer used in Example 7.
[SEQ ID NO: 110]
   It indicates the base sequence of a primer used in Example 7.
[SEQ ID NO: 111]
   It indicates the base sequence of a probe used in Example 7.
[SEQ ID NO: 112]
   It indicates the base sequence of a primer used in Example 7.
[SEQ ID NO: 113]
   It indicates the base sequence of a primer used in Example 7.
[SEQ ID NO: 114]
   It indicates the base sequence of a probe used in Example 7.
[SEQ ID NO: 115]
   It indicates the base sequence of a primer used in Example 3.
[SEQ ID NO: 116]
   It indicates the base sequence of a primer used in Example 3.
[SEQ ID NO: 117]
   It indicates the base sequence of a probe used in Example 3.
[SEQ ID NO: 118]
   It indicates the base sequence of a primer used in Example 3.
[SEQ ID NO: 119]
   It indicates the base sequence of a primer used in Example 3.
[SEQ ID NO: 120]
   It indicates the base sequence of a probe used in Example 3.
[SEQ ID NO: 121]
   It indicates the base sequence of a primer used in Example 3.
[SEQ ID NO: 122]
   It indicates the base sequence of a primer used in Example 3.
[SEQ ID NO: 123]
   It indicates the base sequence of a probe used in Example 3.
[SEQ ID NO: 124]
   It indicates the base sequence of a primer used in Example 3.
[SEQ ID NO: 125]
   It indicates the base sequence of a primer used in Example 3.
[SEQ ID NO: 126]
   It indicates the base sequence of a probe used in Example 3.
[SEQ ID NO: 127]
   It indicates the base sequence of a primer used in Example 3.
[SEQ ID NO: 128]
   It indicates the base sequence of a primer used in Example 3.
[SEQ ID NO: 129]
   It indicates the base sequence of a probe used in Example 3.
[SEQ ID NO: 130]
   It indicates the base sequence of a primer used in Example 3.
[SEQ ID NO: 131]
   It indicates the base sequence of a primer used in Example 3.
[SEQ ID NO: 132]
   It indicates the base sequence of a probe used in Example 3.
[SEQ ID NO: 133]
   It indicates the base sequence of a primer used in Example 3.
[SEQ ID NO: 134]
   It indicates the base sequence of a primer used in Example 3.
[SEQ ID NO: 135]
   It indicates the base sequence of a probe used in Example 3.
[SEQ ID NO: 136]
   It indicates the base sequence of a primer used in Example 3.
[SEQ ID NO: 137]
   It indicates the base sequence of a primer used in Example 3.
[SEQ ID NO: 138]
   It indicates the base sequence of a probe used in Example 3.

The following abbreviations used herein are according to examples now commonly used in the art and their meanings are as follows.
- DMEM :: Dulbecco's Modified Eagle Medium
- MEM :: Minimum Essential Medium
- RT-PCR :: Reverse Transcription Polymerase Chain Reaction
- ATP: : Adenosine Triphosphate
- DMSO :: Dimethyl Sulfoxide
- EDTA :: Ethylenediaminetetraacetate
- BrdU: : 5-Bromo-2'-Deoxyuridine
- PBS: : Phosphate Buffered Saline
- ELISA :: Enzyme-Linked Immunosorbent Assay

The present invention will be more specifically described below with reference to Examples. However, the scope of the present invention is not intended to be limited to such Examples.

### EXAMPLES

### Example 1: Construction of Compound Evaluation System (Embryoid Body Formation)

The cell line D3 (American Type Culture Collection, CRL-1934) was used as ES cells. To maintain the ES cells, mouse fibroblasts (MEF) (purchased from Millipore) whose cell proliferation was terminated by mitomycin C treatment were seeded on a gelatin-coated plate and used as a feeder. After seeding on the feeder, the ES cells were cultured at 37°C under 5% CO₂ using a culture medium made by adding 20% calf fetal serum (FBS, ES cell-qualified, Invitogen), 100 µM 2-mercaptoethanol (Invitrogen), 1 × MEM nonessential amino acid solution (Invitrogen), 1 × antibiotic-antimycotic (Invitrogen), and 1,000 U/ml leukemia inhibitory factor (LIF, Chemicon) to DMEM (Invitrogen) medium. The medium was exchanged every day and subcultures were performed every 3 days using 0.25% trypsin-1 mM EDTA solution (Invitrogen, hereinafter referred to as "trypsin-EDTA solution"), thereby maintaining an undifferentiated state.

For the stabilization and simplification of a compound evaluation system, a large amount of undifferentiated cells from which feeder cells were removed were freeze-preserved by a method to be described below. The cells were first made into a single cell state using the trypsin-EDTA solution, transferred onto a gelatin-coated culture dish, and cultured at 37°C for 90 minutes. Cells having not adhered thereto were recovered to remove mouse fibroblasts (MEF) as a feeder, followed by culture on another gelatin-coated culture dish for further 3 days. Thereafter, cells again made into a single cell state using the trypsin-EDTA solution were seeded on a gelatin-coated culture dish to a cell concentration of 1×10⁵ cells/cm² and cultured for further 24 hours. These cells were dessociated using the trypsin-EDTA solution, suspended at a concentration of 2×10⁶ cells/ml in a cell banker (Nippon Zenyaku Kogyo Co., Ltd.), and freeze-preserved at -80°C. Subsequent experiments were carried out using the freeze-preserved cells.

To form an embryoid body, the freeze-preserved cells were thawed in a constant-temperature bath at 37°C and then seeded on a 96-well spheroid-plate (Sumitomo Bakelite Co., Ltd.) to 1,000 cells per well. Floating culturing was performed for 1 to 6 days using a culture solution made by adding 10% calf fetal serum (FBS, ES cell-qualified, Invitogen), 2 mM GlutaMAX-1 (Invitrogen), 3×10⁻⁴ M monothioglycerol (Sigma), 1 × MEM nonessential amino acid solution (Invitrogen), and 1 × penicillin-streptomycin (Sigma) to DMEM medium. By forming one embryoid body per well using the 96-well spheroid-plate according to the present technique, an embryoid body having a uniform size was formed compared to that for the conventional method involving performing floating culturing on a non-adhesion culture dish to form a non-uniform embryoid body.

Changes in the expression of initial differentiation markers were examined during the formation of an embryoid body by the present technique. The embryoid body was recovered with time and the total RNA was purified using RNeasy96 or RNeasy mini kit (Qiagen). cDNA was synthesized using PrimeScript RT reagent kit (Takara Bio Inc.), followed by performing quantitative RT-PCR to measure the levels of expression of genes: Brachyury, Flk1, Sox17, Sox1, Oct3/4 and Nanog. The sequences of the primers and probes used for the measurement of the genes are shown in Figure 1.

The results of analyzing the levels of expression of the genes Brachyury, Flk1, Sox17, Sox1, Oct3/4 and Nanog are shown in Figure 5. A transient increase in the expression of Brachyury, a primitive streak marker, was observed during day 4 to day 5 of the floating culturing. In addition, the expression of Flk1, a mesoderm marker, and Sox 17, an endoderm marker, also markedly increased from day 4 to day 5 of the floating culturing. The expression of Sox1, an ectoderm marker, also gradually increased during the period of culture. In contrast, the expression of undifferentiation markers Oct3/4 and Nanog gradually decreased as the period of the floating culturing is prolonged. These results demonstrated that an embryoid body having a uniform size is formed by using the method described in this Example and that differentiation of ES cells in the direction of 3 germ layers are induced by the floating culturing of the ES cells for 4 or more days.

### Example 2: Construction of Compound Evaluation System (Transition from Embryoid Body to Adhesive Culture)

To permit high-throughput compound evaluation, an embryoid body is dispersed by a method as described below for adhesion culture. According to the technique described in Example 1, an embryoid body is prepared by performing floating culturing for 4 days and then treated with the trypsin-EDTA solution to prepare single cell state. The cells in a single cell state were seeded onto a gelatin-coated 96-well plate (Asahi Techno Glass Corporation) at 1,000 cells per well and subjected to adhesion culture for 1 to 4 days. The same culture medium as that used for the embryoid body formation was used. The levels of expression of initial differential markers during the adhesion culturing for 1 to 4 days were measured using the same technique as in Example 1. The sequences of primers and probes used for the measurements of the genes are shown in Figure 1.

The results of analyzing the levels of expression of initial differential markers during the adhesion culturing for 1 to 4 days are shown in Figure 6. The levels of expression of Flk1 and Brachyury expressed in the mesoderm were noticeably increased by the adhesion culturing. The level of expression of Sox1, an ectoderm marker, was decreased by the adhesion culturing, while the level of expression of Nestin, a neural precursor cell marker, was gradually increased. The expression of Cdx2, a trophectoderm marker, was also noticeably induced by the adhesion culturing. In contrast, the expression of Foxa2, an endoderm marker, is decreased by the adhesion culturing. These results demonstrated that even after transition from the embryoid body to the adhesion culturing, differentiation into the mesoderm system, the nerve system and the trophectoderm was induced.

Based on the above results, a compound evaluation system using ES cells was set up. The schematic of this evaluation system is shown in Figure 7. The evaluation system is comprised of the procedure which involves (1) allowing the cells to form an embryoid body in a floating culture for 4 days using a 96-well spheroid plate, (2) treating the embryoid body with a trypsin-EDTA solution to prepare single cells of the embryoid body, followed by seeding the cells on a gelatin-coated 96-well plate, (3) adding a compound to be evaluated simultaneously with the seeding of the cells and performing adhesion culturing for 4 days, and (4) measuring the level of expression of each differentiation marker at the end of culture to evaluate the influence of the compound on the differentiation. As for the differentiation markers for use in the measurements, the following seven genes were selected: an undifferentiation marker, Nanog; neural markers, Tubb3 and Nefh; a myocardial marker, Actc1; a smooth muscle marker, Acta2; an endothelial cell marker, Tie2; and a mesoderm marker, Flk1. In addition, the level of expression of Gapdh was also measured as an internal control reflecting the number of living cells. To evaluate the influence of the compound on the number of living cells, the level of expression of Gapdh was also used as one item for evaluation. As for the levels of expression of the other differentiation markers, the values corrected for the level of expression of Gapdh for the evaluation of the compound were used to avoid the influence of an increase or decrease in the number of cells.

### Example 3: Verification of Compound Evaluation System Using Compound Involved in Differentiation

To verify whether the screening method of the present invention is effective in detecting a compound involved in differentiation, changes in the expression of differentiation markers were examined when various compounds reported to be involved in differentiation were each added. Compounds involved in differentiation were evaluated according to the protocol described in Example 2.

As a result, the level of expression of at least one type of gene was found to be increased by 2 times or more with the addition of 1 µM 6-bromoindirubin-3' oxime (BIO, GSK3β inhibitor), 10 µM PD169316 (p38 MAPK inhibitor), 50 nM retinoic acid, 3 µM dexamethasone, 0.5 µM BIX-01294 (G9a histone methyltransferase inhibitor), 1 µM SB 431542 (ALK5 inhibitor), or 1 µM suberoylanilide hydroxamic acid (SAHA, HDAC inhibitor), as compared to that with the addition of DMSO. The pattern of the increase or decrease in the expression of 8 types of genes by the addition of a compound varied with each compound; it was deduced that each compound differentially influenced the differentiation. The activities of various compounds involved in differentiation could be detected by the screening method of the present invention, demonstrating that the screening method of the present invention was very useful in exploring a new a differentiation-regulating agent.

### Example 4: Compound Evaluation Using LOPAC Library

Using the screening method of the present invention, a commercially available compound library, LOPAC¹²⁸⁰ (1,280 compounds, Sigma) was screened for compounds involved in differentiation. According to the protocol described in Example 2, compounds were added to the final concentration of each compound of 3 µM and the concentration of DMSO as a solvent of 0.15%, and subjected to an evaluation. In the screening, to measure the levels of expression of the above-described 8 types of genes in high-throughput, a Multiplex RT-PCR system, which can measure the levels of expression of a plurality of genes at the same time, was set up. The expression of the 8 types of genes was measured by 3 types of Multiplex RT-PCR: set 1 (Gapdh (fluorescent probe BODIPY), Acta2 (fluorescent probe VIC)), set 2 (Actc1 (fluorescent probe BODIPY), Tubb3 (fluorescent probe VIC), Nanog (fluorescent probe FAM)), and set 3 (Flk1 (fluorescent probe BODIPY), Nefh (fluorescent probe VIC), Tie2 ((fluorescent probe FAM)). The sequences of primers and probes used for the measurement of the genes are shown in Figure 1.

As a result of screening, 56 compounds decreased the level of expression of Gapdh to 5% or less relative to a control in which DMSO was added, suggesting that these compounds have cytotoxicity. These 56 compounds were excluded from subsequent analysis. From among the other compounds, compounds were selected which increased the level of expression of at least one gene to 2 times or more that of a control in which DMSO was added. In addition, compounds found to be active were again subjected to the same experiment to examine reproducibility; 52 compounds (4.1% of all of the compounds) for which the reproducibility was confirmed were finally selected as hit compounds (hereinafter sometimes referred to as "selected compounds") (Figure 2). The pharmacological actions of hit compounds are shown in Figure 3. The hit rate for each gene is 2.7% for Flk1, 0.2% for Actc1, 1.6% for Acta2, 0.6% for Tie2, 0.5% for Nefh, 0.4% for Tubb3, 0.0% for Nanog, and 1.1 % for Gapdh. These results demonstrated that the simultaneous measurements of the expressions of 8 types of genes enabled detections of activities of more compounds than those enabled by the measurement of the expression of only 1 gene. In addition, the compounds selected as the hit compounds included compounds such as BIO, PD 169316 and retinoic acid known from other reports to be involved in differentiation and also observed to be active in the compound evaluation system described in this Example. These results show that compounds involved in differentiation was correctly selected by the screening method of the present invention. The hit compounds also included 14 compounds increasing the level of expression of Gapdh. To examine the influence of these compounds on the number of living cells, the amount of ATP in wells was measured using the CellTiter-Glo luminescent cell viability kit (Promega). As a result, the addition of each of the 14 compounds was observed to increase the amount of ATP in a corresponding well. These results demonstrated that these 14 compounds have the action of increasing the number of living cells at least under the present differentiation conditions.

### Example 5: Influence of Compound Selected by Screening on Expression of Other Differentiation Marker

To analyze the influence of the selected compounds on the differentiation of ES cells in further detail, the levels of expression of differentiation markers other than the 8 types of genes used for the screening were also measured using the same technique as that described above. The levels of expression of the undifferentiation markers Oct3/4, Sox2, Klf4 and Akp2, the primitive ectoderm marker Fgf5, the primitive streak markers Brachyury and Snail1, the trophectoderm markers Cdx2 and Bmp4, the marker for mesoderm and endoderm Cxcr4, the marker for extraembryonic endoderm Gata4 and Laminin B1, the neural markers Nestin and p75NTR, the skeletal muscle markers Acta1 and Tpm1, the smooth muscle marker Cnn1, the osteoblast cell marker Opn, the hematopoietic stem cell marker c-kit, and the chondrocyte marker Sox9 as differentiation markers were measured. The sequences of primers and probes used for the measurement of the genes are shown in Figure 1.

Of the selected compounds, 32 compounds were observed to have the activity of increasing the levels of expression of at least 2 types of genes by 2 times or more (Figure 2). To compare the gene expression patterns for these 32 compounds, the expression data were subjected to clustering based on correlation distance.

The results are shown in Figure 11. As a result, it was demonstrated that compounds having the same pharmacological action and similar chemical structures have similar gene expression profiles. For example, thiothixene hydrochloride, perphenazine and cis-(Z)-flupenthixol dihydrochloride which are dopamine receptor antagonists and have similar structures were classified into the same group. PD 169316 and SB 202190 which are p38 MAPK inhibitors and have similar structures were also classified into the same group. In addition, corticosteroids such as hydrocortisone, 21-hemisuccinate sodium, hydrocortisone, betamethasone and beclomethasone, and retinoic acid receptor agonists such as TTNPB, 13-cis-retinoic acid, retinoic acid and retinoic acid p-hydroxanilide were also both classified into the same groups. It was thus demonstrated that the levels of expression of a plurality of differentiation markers could be analyzed using the compound evaluation system of this Example to classify compounds based on the action on differentiation.

### Example 6: Influence of Compound Selected by Screening on Adipose Differentiation of Human Mesenchymal Stem Cell

There was possibility that the selected compounds control the differentiation of adult stem cells as well as ES cells. Accordingly, the influence of these selected compounds was examined on adipose differentiation of human mesenchymal stem cells. The human mesenchymal stem cells were purchased from Lonza and subjected to adhesion culturing using a medium made by addition of 10% calf fetal serum, 2 mM GlutaMAX-1, and 1× penicillin-streptomycin (Sigma) (hereinafter referred to as "growth medium") to low-glucose DMEM (Invitrogen). Upon induction of an adipose differentiation, cells were seeded on a 96-well plate to 3,000 cells per well and then cultured overnight using the growth medium. Thereafter, the adipose differentiation was induced by exchanging the growth medium with an adipose differentiation induction medium (i.e., a medium made by addition of 10% calf fetal serum, 2 mM GlutaMAX-1, 0.5 mM 3-isobutyl-1-methylxanthine (IBMX, Wako Pure Chemical Industries Ltd.), 10 µg/ml insulin (Wako Pure Chemical Industries Ltd.), 1 µM dexamethasone (Wako Pure Chemical Industries Ltd.), 200 µM indomethacin (Wako Pure Chemical Industries Ltd.), and 1× penicillin-streptomycin (Sigma) to high-glucose DMEM (Invitrogen)). Simultaneously with inducing adipose differentiation, each selected compound was added to a final concentration of 3 µM, followed by culture for 5 days. After 5 days, the level of expression of adiponectin as a fat cell marker was measured using the same technique as that described in Example 1. The sequences of primers and probes used for the measurement of the gene are shown in Figure 1.

Among the selected compounds, a ROCK inhibitor Y-27632, a kinase inhibitor HA-100, and a vanilloid receptor antagonist capsazepine were found to be compounds that increase the level of expression of adiponectin (Figure 12a). The action of these compounds to increase the level of expression of adiponectin is equivalent to or stronger than a PPARγ antagonist troglitazone (10 µM).

In addition, the level of expression of PPARγ as a master regulator of adipose differentiation for which the 3 compounds (Y-27632, HA-100 and capsazepine) were each added was also measured using the same technique. As shown in Figure 12a, it was determined that Y-27632 and HA-100 also had the action of increasing the level of expression of PPARγ in a concentration-dependent manner.

The influence of the 3 compounds on fat droplet formation was then examined. Simultaneously with inducing adipose differentiation, 10 µM Y-27632, 10 µM HA-100, or 3 µM capsazepine was added to an adipose differentiation induction medium, followed by adhesion culturing for 5 days. Thereafter, the cells were fixed with 4% paraformaldehyde (Wako Pure Chemical Industries Ltd.) for 30 minutes and then washed 3 times with PBS. In addition, the cells were stained with 0.36% oil red O (Chemicon) at room temperature for 50 minutes and then washed 3 times with distilled water, followed by observing and photographing the cell. As a result, by the addition of Y-27632 or HA-100, it was observed that the amount of fat droplets increased as compared to that for a control in which DMSO was added (Figure 12b). In contrast, no noticeable activity of increasing fat droplet formation was observed for capsazepine.

In addition, when each of the 3 compounds was added, the amount of adiponectin contained in a corresponding culture solution was measured. Simultaneously with inducing adipose differentiation, 10 µM Y-27632, 10 µM HA-100, or 3 µM capsazepine was added, followed by adhesion culture for 8 days. After culture, the amount of adiponectin contained in the culture solution was measured using a human adiponectin ELISA kit (R&D systems). As a result, it was demonstrated that the addition of Y-27632 or HA-100 noticeably increased the amount of adiponectin contained in the culture solution (Figure 12c). In contrast, no action of increasing the amount of adiponectin was observed for capsazepine.
The above results demonstrated that at least Y-27632 and HA-100 have the activity of promoting adipose differentiation of human mesenchymal stem cells. In contrast, 10 compounds decreasing the level of expression of adiponectin to a half or less are present among the selected compounds, suggesting that the selected compounds included compounds suppressing adipose differentiation.

### Example 7: Influence of Compound Selected by Screening on Bone Differentiation of Human Mesenchymal Stem Cell

Then, the influence of the selected compounds was examined on bone differentiation of human mesenchymal stem cells. The human mesenchymal stem cells were maintained and cultured using the same technique as in Example 6. Cells were seeded on a 96-well plate to 1,000 cells per well and then cultured overnight using the growth medium. Thereafter, the bone differentiation was induced by exchanging the medium with a medium made by addition of 10% calf fetal serum, 2 mM GlutaMAX-1, 10 mMβ-glycerophosphate (Wako Pure Chemical Industries Ltd.), 0.05 mM ascorbic acid-2-phosphate (Wako Pure Chemical Industries Ltd.), 0.1 µM dexamethasone (Wako Pure Chemical Industries Ltd.), and 1× penicillin-streptomycin (Sigma) to low-glucose DMEM. Simultaneously with inducing bone differentiation, each selected compound was added to a final concentration of 3 µM, followed by adhesion culture for 8 days. After the end of culture, the level of expression of Alkaline phosphatase (Alpl) as an initial osteoblast marker was measured using the same technique as in Example 1. The sequences of primers and probes used for the measurement of the gene are shown in Figure 1.

As a result, among the selected compounds, 6-bromoindirubin-3'oxime (BIO) as a GSK3β inhibitor and 5-Bromo-2'-deoxyuridine (BrdU) as a thymidine analog was found to be compounds noticeably increasing the level of expression of Alpl (Figure 13a).

The expression of other bone differentiation markers for which BIO or BrdU was added was also examined. Adhesion culture was performed for 8 days after adding BIO or BrdU, followed by measuring the levels of expression of runt-related transcription factor 2 (Runx2), collagen type I alpha 1 (Colla1), and parathyroid hormone receptor (PTHR) using the same technique as in Example 1. The sequences of primers and probes used for the measurement of the genes are shown in Figure 1. As a result, it was demonstrated that 3 µM BIO or 10 µM BrdU also noticeably increased the levels of expression of Runx2, Colla1 and PTHR (Figure 13a).

To further verify the influence of compounds on bone differentiation, staining with alkaline phosphatase was performed. Simultaneously with inducing bone differentiation, 3 µM BIO or 10 µM BrdU was added, followed by adhesion culture for 8 days. After culture, cells were fixed with 4% paraformaldehyde (Wako Pure Chemical Industries Ltd.) for 30 minutes and then washed 3 times with PBS. Alkaline phosphatase kit III (Vector Laboratories) was used according to the appended protocol to stain with alkaline phosphatase. As a result, it was demonstrated that the addition of BIO or BrdU increased the stainability with alkaline phosphatase and these compounds increased alkaline phosphatase activity (Figure 13b).

In addition, the amount of calcium accumulated in cells was examined. Simultaneously with inducing bone differentiation, 3 µM BIO or 10 µM BrdU was added, followed by adhesion culture for 15 days. After culture, cells were washed with PBS, followed by dissolving the cells in a solution consisting of 10 mM Tris-HCl and 0.2% TritonX-100. Using a portion of the solution, the protein concentration was measured employing BCA protein assay kit (Pierce). Hydrochloric acid was added to a final concentration of 0.5 N to the remaining solution, which was then shaken overnight. The amount of calcium dissolving in the solution containing 0.5 N hydrochloric acid was measured using Calcium E-test Kit Wako (Wako Pure Chemical Industries Ltd.). The amount of calcium used the value corrected for the amount of protein for analysis. As a result, the addition of BIO or BrdU increased the amount of calcium in cells (Figure 13c). These results demonstrated that BIO and BrdU had the activity of promoting bone differentiation of human mesenchymal stem cells. In contrast, 2 compounds decreasing the level of expression of Alpl to a half or less are present among the selected compounds, suggesting that the selected compounds included compounds suppressing bone differentiation.

### Example 8: Correlation Between Gapdh Level of expression and ATP Amount in Screening Method of Present Invention

It was examined whether the level of expression of Gapdh measured by the screening method of the present invention was correlated with the number of living cells in the well. According to the method described in Example 2, compounds known to be involved in differentiation, i.e., 1 µM 6-bromoindirubin-3' oxime (BIO), 10 µM PD169316, 10 µM forskolin, 50 nM retinoic acid, 5 µM myoseverin, 3 µM dexamethasone, and 0.5 µM BIX-01294 were each added before adhesion culture for 4 days, followed by measuring the level of expression of Gapdh using the same technique as in Example 1. Simultaneously with the above measurement, the amount of ATP in the well was also measured using a CellTiter-Glo luminescent cell viability kit (Promega). The results are shown in Figure 8. For the addition of any of the compounds, the level of expression of Gapdh was very well correlated with the amount of ATP in the well. These results confirmed that the level of expression of Gapdh was useful as an indicator showing the number of living cells in the well.

### Example 9: Compound Evaluation Using Compound Involved in Differentiation

Example 3 showed that the addition of any of the compounds known to be involved in differentiation changed the expression patterns of the 8 types of genes. The results of these experiments are shown in Figure 9. As described in Example 3, the addition of each compound was found to increase the level of expression of at least one type of gene by 2 times or more compared to the addition of DMSO. The patterns of the increase or decrease in the expression of the 8 types of genes due to the addition of compounds are different for each compound; it was deduced that the compounds differentially influenced differentiation. The activities of various compounds involved in differentiation could be detected by the screening method of the present invention, demonstrating that the screening method of the present invention was very useful in exploring a new differentiation-regulating agent.

### Example 10: Influence of Compound Increasing Gapdh Level of expression on Number of Living Cells

In Example 4, 14 compounds increasing the level of expression of Gapdh were found. Figure 10 shows the results of measurements of the amount of ATP in the well using a CellTiter-Glo Luminescent cell viability kit (Promega) after adding each of these 14 compounds. As described in Example 4, the addition of each of these 14 compounds was observed to increase the amount of ATP in the well. These 14 compounds were demonstrated to have the action of increasing the number of living cells under conditions of the screening method of the present invention.

### Example 11: Influence of Compound Selected by Screening on Differentiation of Human Mesenchymal Stem Cell

Based on the techniques described in Examples 6 and 7, the influence of the selected compounds on adipose differentiation or bone differentiation of human mesenchymal stem cells was examined. Figure 4 collectively shows the influence of the selected compounds on the level of expression of adiponectin during adipose differentiation induction and the influence on the level of expression of Alpl during bone differentiation induction. Compounds Y-27632, HA-100 and capsazepine, which were added upon induction of adipose differentiation, were found to increase the level of expression of adiponectin by 1.3 times or more as compared to a control. Compounds BIO and BrdU, which were added upon induction of bone differentiation, were also found to increase the level of expression of Alpl by 2 times or more as compared to that of a control.

### INDUSTRIAL APPLICABILITY

The screening method of the present invention is useful, for example, for screening for a pharmaceutical product candidate activating stem cells and the like present in a living body to promote regeneration. The pharmaceutical product obtained by screening method of the present invention can be used, for example, to eliminate patient's burdens such as surgery and use of an immunosuppressant. In addition, the screening method of the present invention can be used for efficiently mass-preparing all cells and tissues of human or higher animals; thus, it can be applied to the screening, drug efficacy evaluation, safety studies and the like of candidate drugs in conventional drug discovery processes.

## Claims

1. A method for screening for a substance capable of regulating the regeneration, proliferation or differentiation of a cell or an organ, comprising the following steps (1) to (5):
(1) allowing a cell having a regenerative, proliferative or differentiative capability to form an embryoid body;
(2) treating the embryoid body produced in the step (1) with a digestive enzyme to prepare single cells from the embryoid body;
(3) seeding the cells prepared in the step (2) onto an adhesive plate, and adding a candidate substance to the plate to perform adhesion culturing of the cells on the plate;
(4) conducting quantitative and simultaneous analysis of the levels of expression of at least two types of genes involved in the regeneration, proliferation or differentiation of cells after the adhesion culturing of the step (3); and
(5) evaluating the influence of the candidate substance on the regeneration, proliferation or differentiation of cells based on the results of the quantitative analysis obtained in the step (4).

2. The method according to claim 1, wherein the cell in the step (1) is selected from the group consisting of an embryonic stem cell and an iPS (induced pluripotent stem) cell of a human and a warm-blooded animal.

3. The method according to claim 1, wherein the cell in the step (1) is cultured for a period of 3 to 6 days to form an embryoid body.

4. The method according to claim 1, wherein the cell is selected, as a target of the substance capable of regulating the regeneration, proliferation or differentiation, from the group consisting of an embryonic stem cell and an iPS (induced pluripotent stem) cell of a human and a warm-blooded animal and cells obtained by inducing differentiation of these cells, and a tissue stem cell present in a living tissue or an *in vitro* culture.

5. The method according to claim 1, wherein the substance capable of regulating the regeneration, proliferation or differentiation is a synthetic compound, a natural product, a protein, a peptide, a lipid, an amine, an amino acid, a sugar, a nucleic acid, or an ion.

6. The method according to claim 1, wherein the substance capable of regulating the regeneration, proliferation or differentiation is selected from the group consisting of an agonist and an antagonist of a receptor, a biosynthetic pathway inhibitor, an inhibitor of protein-protein interaction, an inhibitor and a substrate of an enzyme, a coenzyme, an inhibitor and an activator of signal transduction system, an inhibitor and a modulator of channel, a vitamin, an antioxidant, an inhibitor and a promoter of apoptosis, an antiviral agent, a surfactant, an anti-sense oligonucleotide, siRNA, an antibiotic, a compound synthesized by a combinatorial chemistry method, and synthetic intermediates thereof.

7. The method according to claim 1, wherein the cell or the organ targeted by the substance capable of regulating the regeneration, proliferation or differentiation is selected from the group consisting of a spleen cell, a neuronal cell, a glial cell, a pancreatic β cell, a bone marrow cell, a mesangial cell, a Langerhans cell, an epidermal cell, an epithelial cell, an endothelial cell, a fibroblast cell, a fibrocyte, a muscle cell, a fat cell, immune cells, a synovial cell, a chondrocyte, a bone cell, an osteoblast, an osteoclast, a mammary gland cell, a hepatocyte or interstitial cell, or precursor cells thereof, a blood cell-based cell, brain, regions of the brain, spinal cord, hypophysis, stomach, pancreas, kidney, liver, gonad, thyroid, gall-bladder, bone marrow, adrenal gland, skin, muscle, lung, gastrointestinal tract, blood vessel, heart, thymus, spleen, submandibular gland, peripheral blood, a peripheral blood cell, prostate, testicle, testis, ovary, placenta, uterus, bone, joint, and skeletal muscle.

8. The method according to claim 1, wherein the substance capable of regulating the regeneration, proliferation or differentiation is a prophylactic and therapeutic agent for a disease selected from the group consisting of central diseases, inflammatory diseases, circulatory diseases, cancers, diabetes, immune system diseases, liver/gallbladder diseases, alimentary diseases, heat bum, bone fracture, and alopecia.

9. The method according to claim 1, wherein the digestive enzyme in the step (2) is trypsin.

10. The method according to claim 1, wherein the adhesive plate in the step (3) is a gelatin-coated plate having a plurality of wells.

11. The method according to claim 1, wherein the quantitative and simultaneous analysis of the levels of expression of at least two types of genes in the step (4) is performed by Multiplex RT-PCR.

12. The method according to claim 1, wherein the genes involved in the regeneration, proliferation or differentiation of cells in the step (4) are selected from the group consisting of:
(A) undifferentiation markers, Nanog, Oct3/4, Sox2, Klf4 and Akp2;
(B) a primitive ectoderm marker, Fgf5;
(C) primitive streak markers, Brachyury and Snail 1;
(D) trophectoderm markers, Cdx2 and Bmp4;
(E) neural markers, Tubb3, Nefh, Nestin and p75NTR;
(F) a myocardial marker, Actc1;
(G) smooth muscle markers, Acta2 and Cnnl;
(H) an endothelial cell marker, Tie2;
(I) a mesoderm marker, Flk1;
(J) a marker for mesoderm and endoderm, Cxcr4;
(K) markers for extraembryonic endoderm, Gata4 and Laminin B 1;
(L) skeletal muscle markers, Acta1 and Tpm1;
(M) an osteoblast cell marker, Opn;
(N) a hematopoietic stem cell marker, c-kit; and
(O) a chondrocyte marker, Sox9.
